# EUROPEAN PATENT APPLICATION

(11) **EP 1 267 171 A1**
(43) Date of publication of application: **18.12.2002**
(21) Application number: 02291496.4
(22) Date of filing: 14.06.2002
(51) Int. Cl.: G01N 33/68

(54) **Cofactor-based screening method for nuclear receptor modulators**

(30) Priority: 14.06.2001 US 297772 P
(71) Applicant: INSTITUT NATIONAL DE LA SANTE ET DE LA RECHERCHE MEDICALE (INSERM), 75013 Paris (FR)
(72) Inventor: Auwerx, Johan, 67150 Hindisheim (FR); Chambon, Pierre, 67113 Blaesheim (FR); Picard, Fr-d-ric, 67000 Strasbourg (FR)
(74) Representative: Ahner, Francis

(57) **Abstract**

The invention relates to methods that enable identification of modulators of nuclear receptor (NR) activity and to methods for treating and/or preventing diseases or pathologic conditions associated with cell types that express said nuclear receptors. More particularly, the invention concerns to methods enabling the identification of compounds that induce or inhibit the recruitment of the p160 family cofactors, especially TlF-2 and/or SRCs cofactors and that are useful for modulating the PPAR-gamma biological activity and for treating and/or preventing various PPAR-gamma related diseases and conditions, including metabolic or cell proliferative disorders.

## Description

The invention relates to methods that enable identification of modulators of nuclear receptor (NR) activity and to methods for treating and/or preventing diseases or pathologic conditions associated with cell types that express said nuclear receptors. More specifically, it concerns to methods for identifying compounds that modulate the selective recruitment of a particular nuclear receptor cofactor but not other cofactors known to be able to be recruited by said nuclear receptor. In particular, the invention concerns to methods enabling the identification of compounds that induce or inhibit the recruitment of p160 family cofactors and that are useful for modulating the PPAR-gamma biological activity and for treating and/or preventing various PPAR-gamma related diseases and conditions, including metabolic or cell proliferative disorders. More particularly, the invention concerns to methods enabling the identification of compounds that induce or inhibit the recruitment of TIF-2 and/or SRCs cofactors and that are useful for modulating the PPAR-gamma biological activity and for treating and/or preventing various PPAR-gamma related diseases and conditions, including metabolic or cell proliferative disorders.

The following description is provided to aid in understanding the invention but is not admitted to be prior art to the invention.

Peroxisome proliferator-activated receptors (PPARs) are transcription factors that belongs to the nuclear hormone receptor superfamily. These receptors function as ligand-activated transcription factors that control the expression of target genes by binding as heterodimers with the retinoid X receptors (RXRs) to cognate sequences (PPREs) in the promoter regions of those target genes. The first PPAR target genes identified were found to encode mainly enzymes involved in glucose, lipid, and cholesterol metabolism. However, further investigations have shown that PPARs have pleiotropic biological activities and wide-ranging medical applications, extending from uses in the treatment of metabolic disorders to possible applications in the treatment of inflammation and cancer (Spiegelman, 1998, Diabetes, 47, 507-514 ; Schoonjans et al., 1997, Curr. Opin. Lipidol., 8, 159-166). Those skilled in the art will appreciate the numerous additional examples of PPAR mediated diseases and pathologic conditions that have been described in literature (see below). For example, the discovery that these transcription factors are involved in the control of lipid metabolism has provided new insights into the regulation of vertebrate energy homeostasis, and further has provided new molecular targets for the development of therapeutic agents for disorders such as obesity, diabetes and dyslipidemia, cardiovascular disease and related conditions.

The PPAR subfamily includes three subtypes, i.e. PPAR-alpha, PPAR-beta and PPAR-gamma that have distinct tissue expression patterns and exert different physiological roles. PPAR-alpha (PPARα or NR1C1) is highly expressed in the liver, skeletal muscle, kidney and heart, and stimulates the expression of several enzymes involved in peroxisomal beta-oxidation. In addition to being activated by a variety of medium and long-chain fatty acids, PPAR-alpha was found to be the molecular target of the fibrate class of hypolipidemic drugs, such as clofibrate (i.e. 2-(4-chlorophenoxy)-2-methylpropanoic acid ethyl ester), fenofibrate (i.e. 2-(4-(4-chlorobenzoyl)phenoxy)-2-methylpropanoic acid isopropyl ester), bezafibrate (i.e. 2-(4-(4-chlorobenzoylaminoethyl)phenoxy)-2-methylpropanoic acid), ciprofibrate (i.e. 2-(4-(2,2-dichlorocyclopropyl)phenoxy)isobutyric acid) , beclofibrate and etofibrate, as well as gemfibrozil (i.e. 2-(2,4-dimethylphenoxypropyl)-2-methylpropanoic acid ) (Fruchart, 2001, Am. J. Cardiol., 88, 24N-29N). Examples of PPAR-alpha ligands are provided in US 6,071,955.

PPAR-beta (PPARβ or NR1C2; also known as PPAR-delta, NUC-1 or FAAR) is ubiquitously expressed and its role in mammalian physiology is still largely undefined. However, Oliver et al. (2001, Proc. Natl. Acad. Sci., 98, 5306-11) have recently demonstrated that PPAR-beta is implicated in the regulation of reverse cholesterol transport and Michalik et al. (2000, Horm. Res., 54, 263-268) have shown that PPAR-beta is implicated in the control of keratinocyte proliferation and is necessary for rapid healing of a skin wound. The human DNA sequences for the PPAR-beta has been cloned and is fully described in Schmidt et al., 1992, Molecular Endocrinology, 6, 1634-1641, and is herein incorporated by reference.

PPAR-gamma (PPARγ or NR1C3) is most abundantly expressed in adipose tissues, the large intestine, and cells of the monocyte lineage. PPAR-gamma plays a central role in adipogenesis, the regulation of fatty acid storage in adipose tissue, insulin sensitization and in the control of circulating glucose levels. PPAR-gamma has been reported to affect cell proliferation, differentiation (e.g. adipocyte differentiation) and apoptosis pathways. Further evidence is accumulating that suggests an important role for PPAR-gamma in atherosclerosis, inflammation and cancer (for a review, Fajas et al., 2001, J. Mol. Endo., 27, 1-9 or Rosen et al., 2000, Genes & Dev 14, 1293-1307, herein incorporated by reference). PPAR-gamma ligands include prostanoids, fatty acids, thiazolidinediones and N-(2-benzoylphenyl)tyrosine analogues which improve metabolic abnormalities associated with type 2 diabetes, such as hyperglycemia, hyperlipidemia, insulin resistance and other cardiovascular risk factors (Lenhard, 2001, Receptors Channels , 7, 249-58). The DNA sequences for the PPAR-gamma receptors have been described in Elbrecht, et al., 1996, BBRC 224, 431-437, and are herein incorporated by reference (see also reference P37231 of NCBI data base).

Literature provides numerous examples illustrating that PPARs are closely involved in a wide array of diseases or pathological conditions which are associated with cells expressing these nuclear receptors. More specifically, PPARs are useful as drug targets in methods for reducing blood glucose, cholesterol and triglyceride levels and are accordingly explored for the treatment and/or prophylaxis of insulin resistance (type 2 diabetes ; see for example WO 98/05331), impaired glucose tolerance, dyslipidemia, and other disorders related to Syndrome X, also known as Metabolic Disease Syndrome, (WO 97/25042, WO 97/10813, WO 97/28149 ; see also Kaplan et al., 2001, J Cardiovasc Risk, 8, 211-7) including hypertension, obesity, atherosclerosis, thrombosis (Duez et al., 2001, J. Cardiovasc. Risk, 8, 185-186), coronary artery disease and other cardiovascular disorders. Further, PPARs have been shown to be potential targets for the treatment of inflammatory diseases such as cutaneous disorders (including acne vulgaris, cutaneous disorders with barrier dysfunction, cutaneous effects of aging, poor wound healing associated with altered signal transduction ; see Smith et al., 2001, J. Cutan. Med. Surg., 5, 231-43), gastrointestinal diseases (WO 98/43081) or renal diseases including glomerulonephritis, glomerulosclerosis, nephrotic syndrome, hypertensive nephrosclerosis ; similarly PPAR ligands should be useful for improving cognitive functions in neurologic diseases (Landreth and Heneka, 2001, Neurobiol Aging, 22, 937-44) or in dementia, for treating diabetic complications, psoriasis, polycystic ovarian syndrome (PCOS) or for preventing and treating bone loss, e.g. osteoporosis ; or for antiviral, antiproliferative or antitumoral treatments (see for example US 5,981,586 or US 6,291,496).

Thus, it is obvious that the PPARs are exciting targets for the development of therapeutic compounds likely to have utility in the treatment of diseases that involve insulin sensitivity, lipid and glucose homeostasis, as well as vascular or inflammatory diseases or disorders. Although, the responses observed in the context of these various treating and/or preventing methods, are encouraging (for example, the thiazolidinedione (TZD) class of medication, e.g. troglitazone, rosiglitazone or pioglitazone, unambiguously plays a critical role in improving insulin sensitivity in patients with type 2 diabetes ; see Cheng lai and Levine, 2000, Heart Dis., 2, 326-333), they are not fully satisfactory treatments because of the occurrence of undesirable side effects , including weight gain, raise in Low Density Lipoprotein (LDL) cholesterol levels, hypertension, cardiac hypertrophy, haemodilution, liver toxicity, oedema, anemia ; see Haskins et al., 2001, Arch Toxicol. , 75, 425-438 ; Yamamoto et al., 2001, Life Sci., 70, 471-482 ; Scheen, 2001, Diabetes Metab., 27, 305-313 ; Gale, 2001, Lancet, 357, 1870-1875 : Forman et al., 2000, Ann. Intern. Med., 132, 118-121 and Al-Salman et al., 2000, Ann. Intern. Med., 132, 121-124). Thus, it is desirable to have novel improved products and/or novel methods which enable the treatment and/or the prevention of diseases or pathological conditions associated with cell types that express PPAR nuclear receptors. Besides it is widely acknowledged that most of the side effects observed with TZD derivatives are attributable to the full-agonist property of said compounds, thus it is desirable to identify new compounds that are not necessarily full-agonist. Similarly, it is desirable to have methods for identifying, characterizing and/or evaluating said types of products and methods.

It is widely acknowledged that nuclear receptors, such as PPAR-gamma, achieve trancriptional activation or repression by binding to cognate sequences in the promoter regions of target genes and by recruiting numerous cofactor complexes whose activities range from chromatin remodeling, histone and cofactor modification, to basic transcription machinery recruitment (Glass, & Rosenfeld, 2000, Genes Dev., 14, 121-141). These cofactors may to a large extend determine the specificity of the action of nuclear receptors and integrate their action in a network of stimuli whose proper orchestration leads to a specific cellular response. Hence, the determination of the multiple partnerships in which each nuclear receptor is engaged, as a function of time and cell type, is a crucial aspect leading to a better understanding of the activity of nuclear receptors on transcriptional regulation. For instance, it is known that for certain hormones, such as estrogen, the response to the hormone is determined almost to the same extent by the presence of the respective nuclear hormone receptor, as by the presence of the cofactors, which interact with the receptors. Various PPAR cofactors have been identified so far. Some cofactors such as p300/CBP (Dowell et al., 1997, J. Biol. Chem. 272, 33435-33433), SRC-1 (Onate et al., 1995, Science 270, 1354-1357), TIF2 (GRIP-2 ; Chakravarti et al., 1996, Nature, 383, 99-103), SRA (Lanz et al., 1999, Cell, 97, 17-27), AIB-1 (Anzick et al., 1997, Science, 277, 965-968), TRAP220/DRIP205 (i.e. PBP ; Zhu et al., 1997, J. Biol. Chem. 272, 25500-25506 ; Rachez et al., 1999, Nature, 398, 824-828), PGC-1 (Puigserver et al., 1998, Cell 92, 829-839), PRIP (Zhu et al., 2000, J Biol Chem 275, 13510-13516), PGC-2 (Castillo et al., 1999, Embo J , 18, 3676-3687), ARA70 (Heinlein et al., 1999, J Biol Chem 274, 16147-16152), RIP140 (Treuter et al., 1998, Mol Endocrinol 12, 864-881), enhance their transcriptional activity, whereas SMRT (Lavinsky et al., 1998, Proc. Natl. Acad. Sci. USA 95, 2920-2925) and N-CoR (Dowell et al., J Biol Chem 274, 15901-15907) repress it. Additionally, it has been shown that members of the PPAR-gamma cofactor family (e.g. the 160-kDa protein (SRC-1/TIF2/AIB-1), CBP/p300 or TRAP220/DRIP205) interact directly with PPAR-gamma and potentiate nuclear receptor transactivation function in a ligand-dependent fashion leading to biological action or side effects that can differ according to the ligand used (Adams et al., 1997, J. Clin. Invest., 100, 3149-3153). Kodera et al, (2000, J Biol Chem., 275, 33201- 33204) have examined whether interactions between PPAR-gamma and known cofactors were induced to the same extent by different classes of PPAR-gamma ligands (natural and synthetic) and concluded that the overall structure of PPAR-gamma and cofactors complexes may be different according to the ligands involved, resulting in the activation of a particular set of target gene promoters that exert different biological actions.

Nevertheless, in spite of this large amount of information related to nuclear receptor cofactors, no specific results have been reported to date that establish a direct link between at least one of said cofactor and the above mentioned side effects observed, for exemple, with PPAR ligands. Among the side-effects reported for the TZD drugs, rosiglitazone and troglitazone, it is clear that the stimulation of adipocyte differentiation, which translates into weight gain, is a highly unwanted feature for a drug that is used in the treatment, for example, of type 2 diabetes which is a disease associated with and aggravated by obesity.

Thus, the general problem underlying the invention is to develop selective modulators of nuclear receptor activity, such as PPAR-gamma (PPARγ). In preferred embodiment, the modulator activity is preferably targeted to a particular tissue, and do not affect nuclear receptor function in other tissues. In even more preferred embodiment, the modulator of the nuclear receptor activity identified according to the present invention are able to modulate energy fluxes in adipose tissues, said modulation resulting in either lower body weight and insulin sensitization, or enhancement of the body fat mass.

The p160 family of cofactors, composed of SRC-1, TIF2 and SRC-3, is of notable interest for PPAR-gamma. SRC-1 was initially isolated as a progesterone receptor (PR) coactivator (Onate et al., 1995, Science 270, 1354-1357) but has later also been shown to interact in a yeast two-hybrid system with the PPAR-gamma Ligand Binding Domain (Zhu et al, 1996, Gene Expression 6, 185-195). Like CBP/p300, SRC-1 has an intrinsic histone acetyltransferase activity (Spencer et al.,1997, Nature 389, 194-198) and is broadly expressed albeit at different levels (Onate etal., 1995, supra ; Misiti et al., 1998, Endocrinology , 139, 2493-2500). SRC-1 has been shown to have two PPAR binding domains, each containing the LXXLL consensus receptor interaction motif (Heery et al., 1997, Nature 387, 733-736). The related members of the p160 family of cofactors, TIF2 (Voegel et al., 1998, EMBO J. 17, 507-519) and SRC-3 (Torchia, et al., 1997, Nature 387, 677-684), have been shown to also interact with PPAR-gamma in a manner similar to SRC-1.

The Applicant has now investigated whether these different p160 family members can be specifically recruited by different nuclear receptor ligands and whether these different p160 family members exert distinct effects on the regulation of the transcriptional activity of said nuclear receptors and, in consequence, have different biological activity. More specifically, the Applicant proposes a method that allows identification of compounds that are able to induce or inhibit the recruitment of a given cofactor to a particular nuclear receptor, thereby allowing a very fine tuned regulation which results in the activation of specific metabolic processes as well as the elimination of unwanted side effects.

According to a first embodiment, the present invention concerns a method of screening for compounds that modulate the interaction of at least one nuclear receptor with all or part of at least one nuclear receptor cofactor of the p160 family, the method comprising:
(i) incubating a reaction mixture comprising :
   - at least one nuclear receptor, fragments thereof, equivalents or chimeric proteins thereof,
   - at least one potential binding modulator and,
   - at least one nuclear receptor cofactor of the p160 family, fragments thereof, equivalents or chimeric proteins thereof, and
(ii) determining whether the binding to said nuclear receptor of said nuclear receptor cofactor, of said fragment or of said chimeric protein, is increased or decreased in comparison to an assay which lacks the potential binding modulator.

According to the present invention, the "nuclear receptor cofactor of the p160 family" means a cofactors family composed of SRC-1, TIF2 and SRC-3.

According to another embodiment, the present invention concerns a method of screening for compounds that modulate the interaction of at least one nuclear receptor with all or part nuclear receptor cofactor TIF-2, the method comprising:
(i) incubating a reaction mixture comprising :
   - at least one nuclear receptor, fragments thereof, equivalents or chimeric proteins thereof,
   - at least one potential binding modulator and,
   - nuclear receptor cofactor TIF2, fragments thereof, equivalents or chimeric proteins thereof, and
(ii) determining whether the binding to said nuclear receptor of said nuclear receptor cofactor, of said fragment or of said chimeric protein, is increased or decreased in comparison to an assay which lacks the potential binding modulator.

According to another embodiment, the present invention concerns a method of screening for compounds that modulate the interaction of at least one nuclear receptor with all or part of nuclear receptor cofactor SRC-1, the method comprising:
(i) incubating a reaction mixture comprising :
   - at least one nuclear receptor, fragments thereof, equivalents or chimeric proteins thereof,
   - at least one potential binding modulator and,
   - nuclear receptor cofactor SRC-1, fragments thereof, equivalents or chimeric proteins thereof, and
(ii) determining whether the binding to said nuclear receptor of said nuclear receptor cofactor, of said fragment or of said chimeric protein, is increased or decreased in comparison to an assay which lacks the potential binding modulator.

According to another embodiment, the present invention concerns a method of screening for compounds that modulate the interaction of at least one nuclear receptor with all or part of nuclear receptor cofactor SRC-3, the method comprising:
(i) incubating a reaction mixture comprising :
   - at least one nuclear receptor, fragments thereof, equivalents or chimeric proteins thereof,
   - at least one potential binding modulator and,
   - nuclear receptor cofactor SRC-3, fragments thereof, equivalents or chimeric proteins thereof, and
(ii) determining whether the binding to said nuclear receptor of said nuclear receptor cofactor, of said fragment or of said chimeric protein, is increased or decreased in comparison to an assay which lacks the potential binding modulator.

According to the present invention, the term "nuclear receptor" designates a specific molecule, e.g., protein, within a cell that recognizes and binds to other specific molecules, and more specifically to targeted nucleic acid sequences. In preferred embodiment, the nuclear receptor of the invention is a transcription factor, and more preferably it belongs to the nuclear hormone receptor superfamily. More preferably, the nuclear receptor represents a member of the superfamily of structurally related ligand-activated transcription factors, which control the expression of target genes and thus regulate diverse biological activities like growth, development, and homeostasis.

In special embodiment, the reaction mixture comprises all or part of said nuclear receptor. More specifically, said reaction mixture comprises at least one fragment of said nuclear receptor. In preferred embodiments, said fragment will be selected in the full length nuclear receptor sequence and will retain the recognizing and binding properties of the parental full length nuclear receptor regarding other specific molecules, and more specifically targeted nucleic acid sequences. In one embodiment, said fragment is including both the DNA binding domain (i.e. DBD) and the ligand binding domain (i.e. LBD) ; these terms are widely used in literature and are fully understandable by the one skilled in the art. According to one embodiment, the reaction mixture comprises a fragment of the nuclear receptor including the C-terminal domain of said receptor, and in more specific embodiment said fragment of the nuclear receptor comprises the E domain (i.e. ligand binding LBD).

According to a special embodiment, the nuclear receptor of the invention belongs to subfamily of the peroxisome proliferator-activated receptors (PPARs), and more preferably it is PPAR-gamma. The term "PPAR" designates the transcription factors peroxisome proliferator-activated receptors as defined above. In special embodiments, it is selected from the group of PPAR-gammal and PPAR-gamma2. According to special embodiment, the reaction mixture of the invention comprises at least one fragment of PPAR that contains the A, B, C, D and/or E domain of PPAR-gamma. In special embodiment, it comprises combinations of at least two of these domains, for example the AB domain (AB) and/or the DE domain (DE) of PPAR-gamma. In another embodiment, the reaction mixture of the invention comprises at least one fragment of PPAR that contains the A, B, C, D and/or E domain of PPAR-gammal and/or the A, B, C, D and/or E domain of PPAR-gamma2. In preferred embodiments, the reaction mixture of the invention comprises at least one fragment of PPAR that contains at least the E domain of PPAR-gamma (Greene et al., 1995, Gene Expression, 4, 281-299). In one embodiment, said PPAR-gamma protein or fragment is of human origin (SEQ ID NO: 06 - see also reference P37231 of NCBI data base). According to preferred embodiments, the PPAR fragment contains residues 229 to 505 or residues 203-477 of human PPAR-gamma (SEQ ID NO: 06). Alternatively, it is defined from PPAR-gamma of rat (O88275), from pig (O62807), chicken (AAL85323), mouse (P37238), bovine (O18971). These NCBI references and their contents are incorporated herein by reference.

According to another embodiment, the nuclear receptor of the invention is selected from the group consisting of RXRα, TR, RAR, ER. GR. PR, LXRα, LXRβ, Rev-Erba, and ERRa ; factors present in adipose tissues, notably selected from the C/EBP family of transcription factors, ADD-1/SREBP-1 and other adipocyte-related transcription factors, including, but not limited to, STAT1, STAT3, STAT5a, STAT5b, AEBP-1, GATA-2, GATA-3, COUP-TF, HMGI-C, HMGI-(Y), and fragments thereof.

According to the present invention, the terms "nuclear receptor cofactor" designates a molecule, e.g., protein, within the cell that recognizes and binds (i.e. interacts) to the nuclear receptor, said interaction results in the recruitment of other cofactors or other compounds (e.g. ligands) by the nuclear receptor, and facilitates the recognition and binding of the nuclear receptor to its target molecule (e.g. targeted nucleic acid sequences), and/or in modulation of transcriptional effect of the nuclear receptor. Alternatively, the ligand is first binding to the nuclear receptor and thereby facilitates binding of the nuclear receptor cofactor. The cofactors include both so called coactivators and corepressors. Alternatively, the binding of the nuclear receptor cofactor is modulated (inhibited or enhanced) by compounds, such as ligands.

In special embodiment, the reaction mixture comprises all or part of said nuclear receptor cofactor.

The terms "nuclear receptor cofactor TIF-2" designates the human nuclear receptor coactivator 2 as set forth in SEQ ID NO: 01 (see also Genbank accession number XP011633). TIF-2 is also named GRIP1 or NcoA-2 (Chakravarti et al., 1996, Nature, 383, 99-103). Voegel et al. (1998, EMBO J. 17. 507-519) have demonstrated by transient transfection assays with a GAL4 reporter plasmid and chimeras containing various TIF-2 fragments linked to the GAL4 DBD (DNA Binding Domain) the presence of two autonomous transcription activation domains in the C-terminal 460 amino acids of TIF-2. Said domains have been termed AD1 and AD2. The N-terminal AD1 (amino acids 1010-1131) showed a stronger activity than the C-terminal AD2 (amino acids 1288-1464). "Fragment or part of TIF-2" means a polypeptide comprising at least 7, 8, 9 or 10 consecutive amino acids of a TIF-2 polypeptide as set forth in SEQ ID NO: 01. "Equivalent of a TIF-2" means for example a sequence having at least 75 %, 80% or 90% identity with the sequence as set forth in SEQ ID NO: 01.

The terms "nuclear receptor cofactor SRC-1" designates the human human steroid receptor coactivator-one (i.e. SRC-1) as set forth in SEQ ID NO: 03. SRC1 properties have been exposed above. "Fragment or part of SRC-1" means a polypeptide comprising at least 7, 8, 9 or 10 consecutive amino acids of a SRC-1 polypeptide as set forth in SEQ ID NO: 03. "Equivalent of a SRC-1" means for example a sequence having at least 75 %, 80% or 90% identity with the sequence as set forth in SEQ ID NO: 03.

The terms "nuclear receptor cofactor SRC-3" designates the human human steroid receptor coactivator-three (i.e. SRC-3) as set forth in SEQ ID NO: 07. SRC-3 properties have been exposed above. "Fragment or part of SRC-3" means a polypeptide comprising at least 7, 8, 9 or 10 consecutive amino acids of a SRC-3 polypeptide as set forth in SEQ ID NO: 07. "Equivalent of a SRC-3" means for example a sequence having at least 75 %, 80% or 90% identity with the sequence as set forth in SEQ ID NO: 07.

According to the present invention, "percent identity" means the percentage of identical amino acids or nucleotides between two sequences when compared according to the best alignments, this percentage being statistical and the differences between the two sequences being at random and on the total length of the sequences. Optimal alignments of sequences can be achieved by means of the algorithm of Smith Waterman (1981) [Ad. App. Math. 2 : 482], with the algorithm for local homology described in Neddleman and Wunsch (1970) [J. Mol. Biol. 48 : 443], with the similarity search method described by Lipman (1988) [Proc. Natl. Acad. Sci. USA 85 : 2444], with softwares using GAP, BESTFIT, FASTA and TFASTA algorithms available in Wisconsin Genetics Software Package, Genetics Computer Group, 575 Science Dr., Madison, WI and DNASIS, Version 2.5 for Windows; Hitachi Software Engineering Co., Ltd, South San Francisco, CA, by using the standard parameters described in the manufacturer brochure. Another possibility is to use the BLAST or FASTDB programs available at WWW.ncbi.nlm.nih.gov with the following parameters "Mismatch penalty 1.00; Gap Penalty 1.00; Gap Size Penalty 0.33; joining penalty 30.0. These algorithms are displayed in Current Methods in Sequencing and synthesis Methods and Applications, pages 127-149, 1988, Ala R. Liss, Inc".

According to another embodiment, the present invention concerns a method of screening for compounds that modulate the interaction of at least one nuclear receptor with a specific selection of nuclear receptor cofactor, wherein the method comprises the following steps :
(a) testing of the modulatory effect of at least one potential binding modulator on the interaction of at least one nuclear receptor with all or part of nuclear receptor cofactor TIF-2 by incubating a reaction mixture comprising :
   - at least one nuclear receptor, or fragments thereof,
   - at least one potential binding modulator and,
   - all or part of nuclear receptor cofactor TIF-2 or at least one chimeric protein including all or part of TIF-2, or fragments or equivalents thereof,
(b) testing of the modulatory effect of the same potential binding modulator on the interaction of the same nuclear receptor with all or part of at least one nuclear receptor cofactor different from TIF-2 by incubating a reaction mixture comprising :
   - the same nuclear receptor, or fragments thereof, as in step (a)
   - the same potential binding modulator as in step (a) and,
   - all or part of at least one nuclear receptor cofactor or at least one chimeric protein, or fragments or equivalents thereof, said nuclear receptor cofactor/chimeric protein being different from the one implemented in step (a),
(c) determining for test (a) and test (b) whether the binding of the respective nuclear receptor cofactor, or chimeric protein, to the respective nuclear receptor is increased or decreased in comparison to an assay which lacks the potential binding modulator, and
(d) comparing the modulatory effects of the tested potential binding modulator observed in test (a) and test (b).

According to another embodiment, the present invention concerns a method of screening for compounds that modulate the interaction of at least one nuclear receptor with a specific selection of nuclear receptor cofactor, the method comprising the following steps :
(a) testing of the modulatory effect of at least one potential binding modulator on the interaction of at least one nuclear receptor with all or part of nuclear receptor cofactor SRC-1 by incubating a reaction mixture comprising :
   - at least one nuclear receptor, or fragments thereof,
   - at least one potential binding modulator and,
   - all or part of nuclear receptor cofactor SRC-1 or at least one chimeric protein including all or part of SRC-1, or fragments or equivalents thereof,
(b) testing of the modulatory effect of the same potential binding modulator on the interaction of the same nuclear receptor with all or part of at least one nuclear receptor cofactor different from SRC-1 by incubating a reaction mixture comprising :
   - the same nuclear receptor, or fragments thereof, as in step (a)
   - the same potential binding modulator as in step (a) and,
   - all or part of at least one nuclear receptor cofactor or at least one chimeric protein, or fragments or equivalents thereof, said nuclear receptor cofactor/chimeric protein being different from the one implemented in step (a),
(c determining for test (a) and test (b) whether the binding of the respective nuclear receptor cofactor, or chimeric protein, to the respective nuclear receptor is increased or decreased in comparison to an assay which lacks the potential binding modulator, and
(d) comparing the modulatory effects of the tested potential binding modulator observed in test (a) and test (b).

According to special embodiments, the test (b) is individually and independently reproduced using different nuclear receptors cofactors. In preferred embodiments, said nuclear receptors implemented in the test (b) are cofactors previously identified and characterized. In preferred embodiments, the nuclear receptor cofactor implemented in the test (b) is selected from the group consisting of p300/CBP (Dowell et al., 1997, *supra*), TIF-2 (Chakravarti et al., 1996, Nature, 383, 99-103), SRC-1 (Onate et al., 1995, *supra*), SRC-3, SRA (Lanz et al., 1999, *supra*), AIB-1 (Anzick et al, 1997, *supra*), TRAP220/DRIP205 (PBP ; Zhu et al., 1997, *supra* , Rachez et al., 1999, *supra*), PGC-1 (Puigserver et al., 1998, *supra*), PRIP (Zhu et al., 2000, *supra*), PGC-2 (Castillo et al., 1999, *supra*), ARA70 (Heinlein et al., 1999, *supra*), RIP140 (Treuter et al., 1998, *supra*), SMRT (Lavinsky et al., 1998, *supra*), p300/CBP (Demarest et al., 2002, Nature, 415, 549), P/CAF (Chen et al., 1997, Cell, 90, 569), CARM1 (Chen et al., 1999, Science, 284, 2174), PARM1 (Koh et al., 2000, J.Biol. Chem., 276, 1089), ACTR (Chen et al., 1997, Cell 90, 569-580) and N-CoR (Dowell et al., *supra*).

According to one special embodiment, the present invention further concerns a method comprising the step of:
(i) incubating a reaction mixture comprising :
   - at least one nuclear receptor, fragment, equivalent or chimeric protein thereof,
   - at least one potential binding modulator and,
   - at least one nuclear receptor cofactor TIF-2, fragment, equivalent or chimeric protein thereof,
   and
   (ii) incubating :
      (a) a reaction mixture comprising :
         - the same nuclear receptor, fragment, equivalent or chimeric protein thereof, as in step (i),
         - the same potential binding modulator as in step (i) and,
         - at least one nuclear receptor cofactor SRC-1, fragment, equivalent or chimeric protein thereof,
            and /or
      (b) a reaction mixture comprising :
         - the same nuclear receptor, fragment, equivalent or chimeric protein thereof, as in step (i),
         - the same potential binding modulator as in step (i) and,
         - at least one nuclear receptor cofactor SRC-3, fragment, equivalent or chimeric protein thereof,
            and /or
      (b) a reaction mixture comprising :
         - the same nuclear receptor, fragment, equivalent or chimeric protein thereof, as in steps (i),
         - the same potential binding modulator as in step (i) and,
         - at least one nuclear receptor cofactor PGC-1, fragment, equivalent or chimeric protein thereof,
         and
   (iii) determining whether the binding to said nuclear receptor of said nuclear receptor cofactors, of said fragments, of said equivalent or of said chimeric proteins, is increased or decreased in comparison to assays which lack the potential binding modulator.

According to another special embodiment, the present invention further concerns a method comprising the step of:
(i) incubating a reaction mixture comprising :
   - at least one nuclear receptor, fragment, equivalent or chimeric protein thereof,
   - at least one potential binding modulator and,
   - at least one nuclear receptor cofactor SRC-1, fragment, equivalent or chimeric protein thereof,
   and
   (ii) incubating a reaction mixture comprising :
      - the same nuclear receptor, fragment, equivalent or chimeric protein thereof, as in steps (i),
      - the same potential binding modulator as in step (i) and,
      - at least one nuclear receptor cofactor PGC-1, fragment, equivalent or chimeric protein thereof,
   and
   (iii) determining whether the binding to said nuclear receptor of said nuclear receptor cofactors, of said fragments, of said equivalent or of said chimeric proteins, is increased or decreased in comparison to assays which lack the potential binding modulator.

According to another special embodiment, the present invention further concerns a method comprising the step of:
(i) incubating a reaction mixture comprising :
   - at least one nuclear receptor, fragment, equivalent or chimeric protein thereof,
   - at least one potential binding modulator and.
   - at least one nuclear receptor cofactor SRC-3, fragment, equivalent or chimeric protein thereof,
   and
(ii) incubating a reaction mixture comprising :
   - the same nuclear receptor, fragment, equivalent or chimeric protein thereof, as in steps (i),
   - the same potential binding modulator as in step (i) and,
   - at least one nuclear receptor cofactor PGC-1, fragment, equivalent or chimeric protein thereof,
   and
(iii) determining whether the binding to said nuclear receptor of said nuclear receptor cofactors, of said fragments, of said equivalent or of said chimeric proteins, is increased or decreased in comparison to assays which lack the potential binding modulator.

The terms "nuclear receptor", "nuclear receptor cofactor", "nuclear receptor cofactor TIF-2", "nuclear receptor cofactor SRC-1" and "nuclear receptor cofactor SRC-3" are as defined above.

The terms "nuclear receptor cofactor PGC-1" designates the human PPAR gamma coactivator-1 as set forth in SEQ ID NO:05 (Esterbauer et al., 1999, Genomics, 62, 98-102). This transcriptional cofactor is implicated in mitochondrial biogenesis and adaptive thermogenesis. Actually, Puigserver et al, (1998, Cell 92, 829-839) have reported that in mouse PGC-1 enhances the expression of uncoupling protein-1, a key mediator of thermogenesis in brown adipose tissue (see also, Tsukiyama-Kohara et al., 2001, Nature Medecine, 7, 1128-1132). "Fragment or part of PGC-1" means a polypeptide comprising at least 7, 8, 9 or 10 consecutive amino acids of a PGC-1 polypeptide as set forth in SEQ ID NO: 05. "Equivalent of a PGC-1" means for example a sequence having at least 75 %, 80% or 90% identity with the sequence as set forth in SEQ ID NO: 05.

The nuclear receptor and nuclear receptor cofactors of the invention are polypeptides. Said polypeptides are preferably recombinant polypeptides. The terms "recombinant polypeptides" relate to any molecule having a polypeptidic chain that can be produced by genetic engineering, through transcription and translation, of a corresponding DNA sequence under the control of appropriate gene regulatory elements within an efficient cellular host (for example SEQ ID NO:02 and SEQ ID NO: 04 for TIF2 and SRC1, respectively). It should be noticed that the man skilled in the art, based on the amino acid sequence of polypeptides and genetic code, can easily determine nucleic acid sequence encoding the corresponding amino acid chain. The expression "recombinant polypeptides" such as those used herein does not exclude the possibility for the polypeptides to comprise of other groups, such as glycosylated groups. The term "recombinant" indeed means that the polypeptide has been produced by genetic engineering, particularly because it results from the expression in a cellular host of the corresponding nucleic acid sequences which have previously been introduced into an expression vector used in said host. Nevertheless, it must be understood that the polypeptides implemented according to the invention can be produced by a different process, for instance by classical chemical synthesis according to methods used in the protein synthesis or by proteolytic cleavage of larger molecules.

According to special embodiments, the free reactive functions which are present in some of the amino acids of the polypeptides (including nuclear receptor and/or nuclear receptor cofactors and/or related fragment thereof) implemented according to the invention, particularly the free carboxyl groups which are carried by the groups Glu and Asp or by the C-terminal amino acid on one hand and/or the free NH₂ groups carried by the N-terminal amino acid or by amino acids inside the peptidic chain, for instance Lys, on the other hand, can be modified in so far as this modification does not alter the above mentioned properties of the polypeptide. The above mentioned carboxyl groups can be acylated or esterified. Other modifications are also part of the invention.

According to specific applications of the invention, it would be desirable to modify or label the polypeptide implemented in the methods in order to allow their detection or in order to visualize their interaction with other compounds (e.g. heterologous proteins). Methods for modifications/labeling polypeptides are well known by those skilled in the art. As an example, please refer to Chapter 18 of Sambrook and Russell (Molecular Cloning ; Third Edition, 2001, Cold Spring Harbor Laboratory Press, Cold Spring Harbor, New York). The polypeptide may be attached to a detectable label.

The invention also relates to methods implementing amino acid sequences constituted by all or part of the above mentioned polypeptides and by a protein or an heterologous sequence with respect to said polypeptide, said protein or heterologous sequence comprising for instance from about I to about 1100 amino acids. These "mixed" amino acid sequences are called "fusion proteins" or "chimeric proteins". Particularly, the amine or carboxyl functions of both terminal amino acids can be themselves involved in the bond with other amino acids. Examples of these "protein or an heterologous sequences" are: (i) epitopes (see Chapter 17, pages 17.90-17.94 of Sambrook and Russell (Molecular Cloning ; Third Edition, 2001, Cold Spring Harbor Laboratory Press, Cold Spring Harbor, New York), and more preferably His-6 epitope recognized by monoclonal antibodies Mabs 6-His, 6xHis and HIS-11, or an epitope selected from the group consisting of substance P, Human c-Myc protein, colicin A protein, influenza virus hemagglutining, SV40 T antigen, FLAG sequence, T7-Tag, AU epitopes, HPOL, Btag, 3b3, IRS, or polypeptide sequences derived there from ; (ii) glutathione-S-transferase or GST protein (Smith and Johnson, 1988. Gene, 67, 31-40).

Furthermore, any peptidic sequences resulting from the modification by substitution and/or by addition and/or by deletion of one or several amino acids of the polypeptides according to the invention are part of the invention in so far as this modification does not alter the properties and characteristics of said polypeptides.

According to a specific embodiment, the invention concerns a chimeric protein comprising a polypeptide as described above fused to a heterologous polypeptide, and more specifically to glutathione-S-transferase (GST) or His-6 epitope.

Methods and conditions for screening compounds able to interact with nuclear receptors (e.g. ligands) are widely disclosed in the art : for example, Glickman et al., 2002, J. Biomolecular Screening, 7, 3-10 ; Le Douarin et al., (2001, Methods Mol Biol, 176, 227-48) have disclosed an *in vitro* screening test using the yeast two-hybrid system that is based on the ligand-dependent interaction of two proteins, a hormone receptor and a coactivator ; Zhou et al., (2001, Methods, 25, 54-61) have disclosed a homogeneous time-resolved fluorescence (HTRF) energy transfer technology which is sensitive, homogeneous, and nonradioactive ; Beaudet et al, (2001,Genome Res, 11, 600-8) have disclosed the AlphaScreen™ technology (Packard BioScience) which allows the development of high-throughput homogeneous proximity assays. The full content of these papers is incorporated herein by reference.

Specific examples of said standard procedures available in the art are the Fluorescence Resonance Energy Transfer (FRET), the CoActivator-dependent Receptor Ligand Assay (CARLA) and the GST-pull down assays or two-hybrid assays (see Experimental Section).

The terms "modulate the interaction" or "modulate the binding" are synonymous and can be used interchangeably. This modulating effect is defined by reference to the natural situation, i.e. the natural binding observed when the tested nuclear receptor cofactor, or related chimeric protein, is contacted with the tested nuclear receptor. Modulation refers to an increase or a decrease of said binding when the "potential binding modulator" is present in the reaction mixture. Generally, an increase of said interaction/binding is assimilated to an enhancement and relates to compounds named activators, agonists or partial agonists. On the contrary, a decrease of said interaction/binding is assimilated to an inhibition and relates to compounds named inhibitors or antagonists. These terms are not limited by any specific grade.

According to one special embodiment, the method of the invention concerns a screening method for compounds that inhibit the interaction of said nuclear receptor with said nuclear receptor cofactor TIF2. According to one special embodiment, the method of the invention concerns a screening method for compounds that inhibit the interaction of the nuclear receptor PPAR-gamma with said nuclear receptor cofactor TIF2.

According to the invention, "inhibition" means that the measured interaction (i.e. between said nuclear receptor, or fragment thereof or chimeric derivate with said nuclear receptor cofactors, or fragment thereof or chimeric derivate) is decreased in comparison to assays which lack the potential binding modulator. "Inhibition" is not limited to any specific grade ; it might be total or partial.

According to another special embodiment, the method of the invention concerns a screening method for compounds that enhance the interaction of said nuclear receptor with said nuclear receptor cofactor TIF2.

According to the invention, "enhancement" means that the measured binding (i.e. between said nuclear receptor, or fragment thereof or chimeric derivate with said nuclear receptor cofactors, or fragment thereof or chimeric derivate) is increased in comparison to assays which lack the potential binding modulator. "Enhancement" is not limited to any specific grade.

According to another special embodiment, the method of the invention concerns a screening method for compounds that enhance the interaction of the nuclear receptor PPAR-gamma with said nuclear receptor cofactor TIF2. In this case, said enhancement can result in stimulating adipocyte differentiation, promoting weight gain, and enhancing insulin sensitization.

According to another special embodiment, the method of the invention concerns a screening method for compounds that enhance the interaction of said nuclear receptor with said nuclear receptor cofactor SRC-1.

According to another special embodiment, the method of the invention concerns a screening method for compounds that inhibit the interaction of said nuclear receptor with said nuclear receptor cofactor SRC-1.

According to another special embodiment, the method of the invention concerns a screening method for compounds that enhance the interaction of said nuclear receptor with said nuclear receptor cofactor SRC-3.

According to another special embodiment, the method of the invention concerns a screening method for compounds that inhibit the interaction of said nuclear receptor with said nuclear receptor cofactor SRC-3.

According to another special embodiment, the method of the invention concerns a screening method for compounds that inhibit the interaction of said nuclear receptor with said nuclear receptor cofactor TIF2 and that enhance the interaction of said nuclear receptor with said nuclear receptor cofactor SRC-1. According to one special embodiment, the method of the invention concerns a screening method for compounds that inhibit the interaction of the nuclear receptor PPAR-gamma with said nuclear receptor cofactor TIF2 and that enhance the interaction of said nuclear receptor with said nuclear receptor cofactor SRC-1.

According to another special embodiment, the method of the invention concerns a screening method for compounds that enhance the interaction of said nuclear receptor with said nuclear receptor cofactor TIF2 and that inhibit the interaction of said nuclear receptor with said nuclear receptor cofactor SRC-1. According to one special embodiment, the method of the invention concerns a screening method for compounds that enhance the interaction of the nuclear receptor PPAR-gamma with said nuclear receptor cofactor TIF2 and that inhibit the interaction of said nuclear receptor with said nuclear receptor cofactor SRC-1.

According to another special embodiment, the method of the invention concerns a screening method for compounds that enhance the interaction of said nuclear receptor with said nuclear receptor cofactor PGC-1.

According to another special embodiment, the method of the invention concerns a screening method for compounds that inhibit the interaction of said nuclear receptor with said nuclear receptor cofactor PGC-1.

According to another special embodiment, the method of the invention concerns a screening method for compounds that inhibit the interaction of said nuclear receptor with said nuclear receptor cofactor TIF2, that enhance the interaction of said nuclear receptor with said nuclear receptor cofactor SRC-1 and that enhance the interaction of said nuclear receptor with said nuclear receptor cofactor PGC-1. According to one special embodiment, the method of the invention concerns a screening method for compounds that inhibit the interaction of the nuclear receptor PPAR-gamma with said nuclear receptor cofactor TIF2, that enhance the interaction of said nuclear receptor with said nuclear receptor cofactor SRC-1 and that enhance the interaction of said nuclear receptor with said nuclear receptor cofactor PGC-1.

According to another special embodiment, the method of the invention concerns a screening method for compounds that enhance the interaction of said nuclear receptor with said nuclear receptor cofactor TIF2, that inhibit the interaction of said nuclear receptor with said nuclear receptor cofactor SRC-1 and that inhibit the interaction of said nuclear receptor with said nuclear receptor cofactor PGC-1. According to one special embodiment, the method of the invention concerns a screening method for compounds that enhance the interaction of the nuclear receptor PPAR-gamma with said nuclear receptor cofactor TIF2, that inhibit the interaction of said nuclear receptor with said nuclear receptor cofactor SRC-1 and that inhibit the interaction of said nuclear receptor with said nuclear receptor cofactor PGC-1.

According to another special embodiment, the method of the invention comprises a further step consisting in assessing the effect of the modulation of the binding of said nuclear receptor cofactor(s) to said nuclear receptor on the transcriptional regulation activity of said nuclear receptor.

Methods for analyzing the modulation of transcriptional regulation activity of said nuclear receptor can be examined in a reconstituted system in cell culture (for example see US 5,668,175). Such a system was used to evaluate the compounds of this invention for their interaction with the nuclear receptor. The system for reconstituting ligand-dependent transcriptional control has been developed by Evans et al., 1988, Science, 240, 889-95 and has been termed "co-transfection" or "cis-trans" assay. This assay is described in more detail in U.S. 4,981,784 and US 5,071,773, which are incorporated herein by reference. Also see Heyman et al., Cell, 68: 397-406 (1992). The co-transfection assay provides a method to evaluate the ability of a compound to modulate the transcriptional response initiated by a nuclear receptor. The co-transfection assay is a functional, rapid assay that monitors hormone or ligand activity, is a good predictor of the *in vivo* activity, and can be used to quantitate the pharmacological potency and utility of such ligands in treating various disease states (Berger, et al., 1992, J. Steroid Biochem Molec. Biol., 41: 733-38). Briefly, the co-transfection assay involves the introduction of various plasmids by transient transfection into a mammalian cell : a plasmid which contains a nuclear receptor receptor cDNA (e.g. PPAR gamma or RXR alpha) and directs constitutive expression of the encoded receptor ; a plasmid which contains a sequence encoding one cofactor polypeptide of the invention and directs constitutive expression of the encoded cofactor and a plasmid which contains a cDNA that encodes for a readily quantifiable protein, e.g., firefly luciferase or chloramphenicol acetyl transferase (CAT), alkaline phosphatase (SPAT or SEAP), under control of a promoter containing a PPAR response element (PPRE), which confers dependence on the transcription of the reporter. This assay can be used to accurately measure efficacy and potency of potential binding modulators.

According to another embodiment, the method of the invention comprises a further step consisting of the determination of the cell and/or tissue specificity :
- of the modulation of the binding of said nuclear receptor cofactor to said nuclear receptor, and/or
- of the effect of said modulation on the transcriptional regulation activity of said nuclear receptor.

Cell and/or tissue specificity can be assessed by cell/tissue-profiling. Said profiling consists of the analysis of the modulation of the binding and/or of transcriptional regulation activity of said nuclear receptor in a reconstituted system in many cell culture, wherein said cells are originated from various tissue.

The present invention further concerns a method for identifying a compound that modulates at least one nuclear receptor function comprising a screening method as disclosed above. According to preferred embodiment, said method is intended for identifying a compound that modulates the PPAR-gamma function.

In another aspect, the present invention concerns a compound that modulates the interaction of at least one nuclear receptor with the nuclear receptor cofactor TIF-2 of the invention. According to a special embodiment, said compound inhibits said interaction.

In another aspect, the present invention concerns a compound that modulates the interaction of at least one nuclear receptor with the nuclear receptor cofactor SRC-1 of the invention. According to a special embodiment, said compound enhances said interaction.

According to another embodiment, said compound farther modulates function of at least one nuclear receptor, and preferably said compound enhances function of at least one nuclear receptor.

In a preferred embodiment, said compounds are identified by a screening or identifying method of the present invention. In a special embodiment, it is a PPAR-gamma receptor modulator.

According to one embodiment, the compound of the invention is a modulator selected from the group consisting of nuclear receptor agonists and nuclear receptor partial agonists.

According to the present invention, the term "nuclear receptor agonist" means nuclear receptor ligand which when combined with its respective nuclear receptor increases a reaction typical for the said receptor, e.g., transcriptional regulation activity. In one embodiment, said agonist is a PPAR-gamma agonist, i.e. a PPAR ligand which potentiates, induces or otherwise enhances the transcriptional activity of a PPAR-gamma receptor, e.g., such as by mimicking a natural ligand for the receptor or as measured by an assay known to one skilled in the art, including, but not limited to, the "co-transfection" or "cis-trans" assays described or disclosed in US 4,981,784, US 5,071,773 or Lehmann et al., 1995, J. Biol. Chem., 270, 12953-12956, which are incorporated by reference herein. More specifically, it has been shown that activation of PPAR activity (i.e. activation of PPAR targeted genes transcription) by PPAR agonist results actually from promoted recruitment of coactivator proteins (e.g. the polypeptide of the invention) to the PPAR receptor by said PPAR agonists.

"Nuclear receptor antagonists" have been described elsewhere (see WO 01/17994). The term "PPAR-gamma antagonist" designates a PPAR-gamma ligand that gives greater than 50% inhibition of transactivation achieved by 100 nM rosiglitazone when tested in the cell-based reporter assay as described in WO 01/17994. As general definition, "PPAR antagonist" designates a PPAR ligand which can inhibit the activity of a corresponding PPAR agonist.

The present invention further concerns a composition comprising at least one compound of the invention as disclosed above and a pharmaceutically acceptable carrier or diluent. These pharmaceutical compositions may be prepared by conventional techniques, e.g. as described in Remington, 1995, The Science and Practise of Pharmacy, 19.sup.th Ed. They may appear in conventional forms, for example capsules, tablets, aerosols, solutions, suspensions or topical applications.

Typical compositions of the present invention are associated with a pharmaceutically acceptable excipient which may be a carrier or a diluent or be diluted by a carrier, or enclosed within a carrier which can be in the form of a capsule, sachet, paper or other container. In making the combination products, conventional techniques for the preparation of pharmaceutical compositions may be used. For example, the active compounds will usually be mixed with a carrier or a diluent, or diluted by a carrier or a diluent, or enclosed within a carrier or a diluent which may be in the form of a ampoule, capsule, sachet, paper, or other container. When the carrier serves as a diluent, it may be solid, semi-solid, or liquid material which acts as a vehicle, excipient, or medium for the active compound. The active compounds can be adsorbed on a granular solid container for example in a sachet. Some examples of suitable carriers or diluents are water, salt solutions, alcohols, polyethylene glycols, polyhydroxyethoxylated castor oil, peanut oil, olive oil, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art. In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811. The pharmaceutical compositions of the invention can be sterilized and mixed, if desired, with auxiliary agents, emulsifiers, salt for influencing osmotic pressure, buffers and/or colouring substances and the like, which do not deleteriously react with the active compounds.

The invention also concerns methods for treating and/or preventing diseases or pathologic conditions associated with cell types that express PPAR receptors comprising administering to the patient at least one compound of or a composition of above disclosed.

In preferred embodiment, said methods for treating and/or preventing diseases or pathologic conditions associated with cell types that express PPAR receptors are not associated with side effects, and preferably are not associated with patient weigh gain.

Similarly, the invention refers to the use of at least one compound of the invention for the preparation of a pharmaceutical composition.

More specifically, the invention refers to the use of at least one compound of the invention for the preparation of a pharmaceutical composition which is not associated with weigh gain when administered to patient in need.

It further concerns the use of at least one compound of the invention for the preparation of a pharmaceutical composition for treating and/or preventing diseases or pathologic conditions associated with cell types that express PPAR receptors.

More specifically, the invention refers to the use of at least one compound of the invention for the preparation of a pharmaceutical composition for treating and/or preventing diseases or pathologic conditions associated with cell types that express PPAR receptors, wherein administration to patient in need of said pharmaceutical composition is not associated with weigh gain.

Alternatively, the composition of the present invention further comprises a natural or synthetic PPAR and/or RXR agonist or antagonist.

Naturally occurring ligands that modulate the activity of PPAR, preferably the PPAR-gamma, including but are not limited to, fatty acids such as arachidonic acid derivatives or metabolites such as eicosanoids (e.g. various isomeric forms of 8-hydroxytetraenoic acid) and cyclopentenone prostaglandins (e.g. prostaglandins in the J and A series and their metabolites), long-chain fatty acids and their derivatives, e.g. 9-and 13-cis-hydroxyoctadecadienoic acid (HODE) (Nagy et al., 1998, Cell, 17, 93, 229-240 ; Chinetti et al., 2001, Z Kardiol, 90 Suppl 3, 125-32). Diterpene acids and auronols (e.g. pseudolaric acids A and B) isolated from Pseudolarix kaempferi (Pan et al., 1990, Planta Med, 56, 383-385; Li et al., 1999, J Nat Prod, 62, 767-769) have also been shown to activate PPAR-gamma and are expected to be useful in the practice of this invention. In one embodiment, said natural PPAR ligand is a prostaglandin J2 or delta-12-prostaglandin J2 (PGJ2) metabolite, and more particularly it is 15-deoxy-delta-12,14-prostaglandin J2 [15-deoxy-Delta(12,14)-PGJ(2) or 15d-PGJ2].

Synthetic ligands that modulate the activity of PPAR are for example antidyslipidemic fibrates (e.g. clofibrate, fenofibrate, benzofibrate, ciprofibrate, gemfibrozil), thiazolidine derivatives (e.g. thiazolidinediones), oxazolidine derivatives (e.g. oxazolidinediones), alpha-alkylthio, alpha-alkoxy and carboxylic acid derivatives of thiazolidines and oxazolidines (Hulin et al. 1996, J Med Chem., 39, 3897-3907), N-2-L-tyrosine derivatives (e.g. N-(2-Benzoylphenyl)-L-tyrosine ; Henke et al., 1998, J Med Chem, 41, 5020-5036), FMOC-L-Leucine (WO0200611), phenyl acetic acid derivatives (Berger et al., 1999, J. Biol. Chem., 274, 6718-6725) and indolethiazolidinedione derivatives (Lohray et al., 1998, J Med Chem, 41, 1619-1630).

Compounds disclosed or described in the following articles, patents and patent applications which have RXR agonist activity are incorporated by reference herein: US 5,399,586 and 5,466,861, WO96/05165, WO94/15901, WO93/11755, WO94/15902, WO93/21146, Boehm, et al. 1994, J. Med. Chem. 38, 3146-3155, Boehm, et al. 1994, J. Med. Chem. 37, 2930-2941, Antras et al., 1991, J. Biol. Chem. 1266, 1157-1161. RXR specific agonists include, but are not limited to, 9-cis-retinoic acid, 4-(1-(3,5,5,8,8-pentamethyl-5,6,7,8-tetrahydro-2-naphthyl)-ethenyl)benzoic acid (3-methyl-TTNEB; LGD 1069), LG 100268 (i.e. 2-[1-(3,5,5,8,8-pentamethyl-5,6,7,8-tetrahydro-2-naphthyl)-cyclopropyl]-pyridine-5-carboxylic acid), 4-[(3,5,5,8,8-pentamethyl-5,6,7,8-tetrahydro-2-naphthy)-2-carbonyl]-benzoic acid, ((E)-2-(2-((5,6,7,8-tetra-hydro-3,5,5,8,8-pentamethyl-2-naphthyl)propen-1- yl)-4-thiophenecarboxylic acid) (AGN 191701), 2-(5,6,7,8-tetra-hydro-5,5,8,8-tetramethyl-2-naphthyl)-2-(carboxyphenyl)-1 ,3-dioxolane (SR 11237), 4-(5H-2,3-(2,5-dimethyl-2,5-hemano)-5-methyl-dibenzo(b,e) (1,4)diazepin-11-yl)-benzoic acid (HX600) or thiadiazepin analogs thereof, 3,7,11,15-tetramethyl hexadecanoic acid (phytanic acid), 6-(1-(3,5,5,8,8-pentamethyl-5,6,7,8-tetrahydronaphthalen-2-y])cyclopropyl) nicotinic acid, ALRT 1057 (i.e. 9-cis retinoic acid, 2-(4-carboxyphenyl)-2-(5,6,7,8-tetrahydro-5,5,8,8-tetramethyl-2-naphthalen yl)-1,3-dithiane (SR11203), 4-(2-methyl)-1-(5,6,7,8-tetrahydro-5,5,8,8-tetramethyl-2-naphthalenyl)propenyl)benzoic acid (SR11217), and the like or a pharmaceutically acceptable salt thereof.

Likewise, the compositions of the present invention can further comprise additional agents. Examples of such additional agents are insulin, insulin derivative, insulin secretagogue, insulin sensitizer, or insulin mimetic ; other examples are mitotic inhibitors, alkylating agents, antimetabolites, nucleic acid intercalating agents, topoisomerase inhibitors, agents which promote apoptosis, or agents which increase immune responses to tumors (e.g cytokine chosen from alpha-, beta- and gamma-interferon, interleukins, and in particular IL-2, IL-4, IL-6, IL-10 or IL-12, tumour necrosis factors (TNFs) and colony stimulating factors (for example GM-CSF, C-CSF and M-CSF). Literature provides to the skilled man with numerous examples of such additional agents.

The compounds and compositions of the present invention are specially adapted to cure, improve or prevent one or more symptoms of diseases or pathologic conditions associated with cells types that express PPAR nuclear receptors.

"Diseases or pathologic conditions associated with cells types that express PPAR nuclear receptors" means diseases or pathologic conditions wherein the observed disorder is associated initially with the deregulation, disturbance, hypersensitivity, or malfunctioning of cells expressing PPAR nuclear receptors, preferably PPAR-gamma receptor, or more specifically in which the disease or pathologic conditions is caused by one or more genes that are under the transcription control of PPARs and preferably of PPAR-gamma, or said disease or pathological condition causing genes are posttranlationally modified in response to PPARs. Examples of these cells are those from liver, skeletal muscle, kidney, heart, CNS, adipose tissues, intestine, or cells of the monocyte lineage. In preferred embodiment, said cell type is an adipocyte or pre-adipocyte. Another example is a PPAR-responsive hyperproliferative cell. Examples of diseases or pathologic conditions associated with cells types that express PPAR nuclear receptors are those associated with impaired metabolism of glucose, cholesterol or triglycerides. More specifically, it is insulin resistance, type 2 diabetes, impaired glucose tolerance, dyslipidemia, hypercholesterolemia, hypertriglycidemia, disorders related to the metabolic disease Syndrome X including hypertension, obesity, hyperglycaemia, atherosclerosis, thrombosis, hyperlipidemia, coronary artery disease and other cardiovascular disorders ; renal diseases including glomerulonephritis, glomerulosclerosis, nephrotic syndrome, hypertensive nephrosclerosis ; neurologic diseases or dementia ; inflammatory diseases such as cutaneous disorders (including acne vulgaris, psoriasis, cutaneous disorders with barrier dysfunction, cutaneous effects of aging, poor wound healing), diabetic complications, polycystic ovarian syndrome (PCOS) and bone loss, e.g. osteoporosis ; gastrointestinal diseases ; viral, proliferative cells or tumoral diseases, such as cancers.

In one embodiment, disease or pathologic condition according to the invention is obesity. Obesity is a disease that had become highly prevalent in affluent societies and in the developing world and which is a major cause of morbidity and mortality. It is characterized by a body mass index above 25. Thus there is a strong need for efficient therapy to treat this disease which has been identified as a leading cause of coronary heart disease, type 2 diabetes, stroke, hyperlipidemia, gout, osteoarthritis, reduced fertility and many other psychological and social problems. Additionally, obesity contributes to insulin resistance and diabetes.

In another embodiment, disease or pathologic condition according to the invention is diabetes or insulin resistance. Insulin resistance is manifested by the diminished ability of insulin to exert its biological action across a broad range of concentrations. During early stages of insulin resistance, the body secretes abnormally high amounts of insulin to compensate for this defect. Within developed countries, diabetes mellitus has become a common problem and is associated with a variety of abnormalities including, but not limited to, obesity, hypertension, hyperlipidemia and renal complications. It is now increasingly being recognized that insulin resistance and hyperinsulinemia contribute significantly to obesity, hypertension, atherosclerosis and type 2 diabetes mellitus. The association of insulin resistance with obesity, hypertension and angina pectoris has been described as a syndrome (Syndrome-X ) in which insulin resistance plays the central role. The term "diabetes" refers to all variant forms of diabetes mellitus (DM), including type 1 DM, type 2 DM, gestational diabetes, juvenile diabetes, etc.

In another embodiment, disease or pathologic condition according to the invention is hyperlipidemia. Hyperlipidemia is considered the primary cause of cardiovascular and other peripheral vascular diseases. An increased risk of cardiovascular disease is correlated with elevated plasma levels of LDL (Low Density Lipoprotein) and VLDL (Very Low Density Lipoprotein) as seen in hyperlipidemia. Numerous studies have shown that lowering of plasma triglycerides and total cholesterol, in particular LDL and VLDL and increasing HDL cholesterol leads to a significant reduction of cardiac events.

In another embodiment, disease or pathologic condition according to the invention is lipo-atrophy and/or lipo-dystrophy. According to said embodiment, it would be desirable to gain weight, to increase the fat mass in the treated patient.

In yet another embodiment, "diseases or pathologic conditions associated with cells types that express PPAR nuclear receptors" also include cellular proliferation, growth, differentiation, or migration disorders. As used herein, a "cellular proliferation, growth, differentiation, or cell migration disorders" is a disorder in which a cell increases in number, size or content, in which a cell develops a specialized set of characteristics which differ from that of other cells, or in which a cell moves closer to or further from a particular location or stimulus. The PPAR molecules of the present invention are involved in signal transduction mechanisms, which are known to be involved in cellular growth, differentiation, and migration processes. Thus, the PPAR molecules may modulate cellular growth, differentiation, or migration, and may play a role in disorders characterized by aberrantly regulated growth, differentiation, or migration. Such disorders include cancer, e.g., carcinomas, sarcomas, leukemias, and lymphomas; tumor angiogenesis and metastasis; skeletal dysplasia; hepatic disorders; and hematopoietic and/or myeloproliferative disorders. Exemplary disorders include, but not limited to, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

In other embodiments, the disease or pathologic condition according to the invention is a disorder characterized by aberrant cell growth of PPAR-responsive cells such as hyperplastic or neoplastic disorders arising in adipose tissue, such as adipose cell tumors, e.g., lipomas, fibrolipomas, lipoblastomas, lipomatosis, hibemomas, hemangiomas and/or liposarcomas.

In still other embodiments, the disease or pathologic condition according to the invention is a disorder characterized by aberrant cell growth of PPAR-responsive cells such as hyperplastic or neoplastic disorders of the hematopoietic system, e.g., leukemic cancers.

In another embodiment, disease or pathologic condition according to the invention is an inflammatory disease including, but not limited to, T-lymphocyte activation and other T-lymphocyte-related disorders ; inflammatory cytokine (e.g. TNF-alpha, interleukin (IL)-1-alpha, IL-1-beta, IL-2, IL-6) production ; activation of nuclear factors that promote transcription of genes encoding inflammatory cytokines. Examples of these nuclear transcription factors include but are not restricted to, nuclear factor-kappaB (NF-kappaB), activated protein-1 (AP-1), nuclear factor of activated T cells (NFAT).

Other examples of disease or pathologic condition according to the invention are chronic viral infections (e.g. HIV, CMV, HSV, HBV, HCV infections), neurodegenerative diseases (e.g. Alzheimer's disease, multiple sclerosis, Parkinson's disease), cardiovascular disease (e.g. atherosclerosis, atherogenesis, vascular restenosis, congestive heart failure), diseases or conditions involving hypoxemia and hypoxic stress (stroke, vascular occlusive disease, MI, atherosclerosis, retinitis, retinal vein occlusion, hypoxic retinopathy, macular degeneration).

According to the present invention, the term "patient" means a mammal, e.g., a primate, e.g., a human.

According to another aspect, the invention provides a method of treating and/or preventing nuclear receptors PPAR mediated diseases or conditions in a patient, comprising the step of administering to said individual a pharmacologically effective dose of a compound or composition of the invention. As used herein, "treatment" of an individual includes the application or administration of a compound or product of the invention to an individual, or application or administration of a compound or product of the invention to a cell or tissue from an individual, who has a diseases or disorder, has a symptom of a disease or disorder, or is at risk of (or susceptible to) a disease or disorder, with the purpose to cure, heal, alleviate, relieve, alter, remedy, ameliorate, improve, or affect the disease or disorder, the symptom of the disease or disorder, or the risk of (or susceptibility to) the disease or disorder.

Alternatively, the present invention concerns a method of treating and/or preventing diseases or conditions in a patient, comprising the step of administering to said individual a pharmacologically effective dose of a compound or composition of the invention said administration resulting in improving the clinical status of said patient.

By "pharmaceutically effective dose" is meant an amount of a pharmaceutical compound or composition having a therapeutically relevant effect in the frame of treatment and/or prevention of conditions mediated by PPAR. A therapeutically relevant effect relieves to some extent one or more symptoms of conditions mediated by PPAR in a patient or returns to normal either partially or completely one or more physiological or biochemical parameters associated with or causative of said conditions, e.g. increasing the sensitivity of cellular response to circulating insulin, curing, reducing, or preventing one or more clinical symptoms of PPAR related conditions, including, but not limited to, hyperglycemia, hyperinsulinemia and hypertriglyceridemia. In a preferred embodiment, a pharmaceutically effective dose of a compound or composition means an amount that increases the uptake of glucose by adipose tissue or muscle tissue. In another preferred embodiment, a pharmaceutically effective dose of a compound or composition means an amount that increases the uptake of triglyceride by adipose tissue. The compounds of the invention are effective over a wide dosage range. For example, in the treatment of adult humans, dosages from about 0.05 to about 100 mg, preferably from about 0.1 to about 100 mg, per day may be used. A most preferable dosage is about 0.1 mg to about 70 mg per day. In choosing a regimen for patients it may frequently be necessary to begin with a dosage of about 2 to about 70 mg per day and when the condition is under control to reduce the dosage as low as from about 0.1 to about 10 mg per day. The exact dosage will depend upon the mode of administration, on the therapeutic effect that is intended to be achieved, the form in which the dosage is administered, the subject to be treated and the body weight of the subject to be treated, and the preference and experience of the physician or veterinarian in charge. Dosages and treatment schedules are readily attainable by routine experimentation to those having ordinary skill in this art. Generally, the compounds are dispensed in unit dosage form comprising from about 0.1 to about 100 mg of active ingredient together with a pharmaceutically acceptable carrier per unit dosage.

Toxicity and therapeutic efficacy of the compounds included in the compound or composition of the invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, special care should be taken to design a delivery system that targets such compounds to the site of affected tissue in order to minimize potential damage to uninfected cells and, thereby, leads to a reduction of side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The route of administration of the compound or composition of the present invention may be any route, which effectively transports the active compound to the appropriate or desired site of action, such as oral, nasal, pulmonary, transdermal or parenteral e.g. rectal, depot, subcutaneous, intravenous, intraurethral, intramuscular, intranasal, ophthalmic solution or an ointment, the oral or intratumoral route being preferred.

If a solid carrier is used for oral administration, the composition may be tabletted, placed in a hard gelatin capsule in powder or pellet form or it can be in the form of a troche or lozenge. If a liquid carrier is used, the preparation may be in the form of a syrup, emulsion, soft gelatin capsule or sterile injectable liquid such as an aqueous or non-aqueous liquid suspension or solution. Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

According to another aspect, the invention concerns a method for modulating TIF2 activity in a patient, comprising administration of a compound or a composition of the invention.

More specifically, the invention concerns a method for inhibiting TIF2 activity in a patient, comprising administration of a compound or a composition of the invention.

According to another aspect, the invention concerns a method for modulating SRC-1 and/or SRC-3 activity in a patient, comprising administration of a compound or a composition of the invention.

More specifically, the invention concerns a method for enhancing SRC-1 activity in a patient, comprising administration of a compound or a composition of the invention.

According to special embodiments, said methods can be practiced for treating a metabolic disorders selected from the group consisting of obesity, cachexia, lipo-atrophy and lipo-dystrophy, lipoma and liposarcoma, type 2 diabetes, insulin resistance, atherosclerosis, hyperlipidemia, hypertension and chronic inflammatory disorders.

The physiological response associated with the administration of a compound or a composition of the invention can be assessed by a method comprising the transplantation or injection of cells which are engineered to express (i) a TIF2 polypeptide or another member of the SRC family such as SCR-1 or ACTR (ii) a PPAR-gamma polypeptide into an animal host or by genetically engineering an animal host using transgenic technologies to carry complex (i) and (ii) and scoring for the formation of said complex in this animal host *in vivo* in presence or absence of the compounds to be tested. In such a case, the polypeptide can be labelled for example using fused fluorescent proteins such as GFP, CFP and YFP. Alternatively, the method can comprise the steps consisting in :
- incubating cells transfected with a vector expressing a TIF2 polypeptide or another polypeptide such as SCR-1, SCR-3, PGC-1 or ACTR with at least one compound to be tested; and growing said cells under condition permissive for differentiation,
- observing the presence or the absence of accumulation of lipid droplets after for example 10 days of culture,
- and identifying compounds which so disrupt, interfere with, potentiate or inhibit said accumulation of lipid droplets. Cells can be selected from the group consisting of primary adipocytes, primary preadipocytes, SGBS, NIH-3T3; 3T3-L1; 3T3 F442A; C3H10-1/2; Swiss 3T3; 3T3 C2 and ob 1771.

Similarly, transgenic mammals in which the gene encoding the cofactor has been disrupted (also described as "cofactor -/-" or "cofactor KO" ; e.g. "TIF-2 -/-", "SRC-I -/" or "SRC-3 -/-") using standard techniques known in the art can be obtained to analyze the physiological response of the nuclear receptor, preferably of the PPAR-gamma, modulator on, for example, plasma glucose/insulin levels, or glucose tolerance, glucose uptake, ketone bodies, rectal body temperature, adipocyte differentiation, whole body weight gain, organ weight gain, including white adipose tissue, brown adipose tissue, liver, kidney, pancreas, spleen, brain, total plasma cholesterol, LDL- cholesterol, HDL-cholesterol, VLDL- cholesterol, plasma triglyceride levels, cardiac hypertrophy, plasma volume, blood pressure, liver function, hematocrit, oedema, etc... This method of analysis is of particular interest in the context of pre-clinical development of drugs. Such single KO mice can also be bred with other KO mice in which other members of the SRC family have been deleted. In preferred embodiments, said mammals are selected among the rodent group, and more preferably it is a transgenic mouse.

TIF-2 -/- and SRC-1-/- KO mice are available. They have been previously constructed and disclosed by Gehin et al, 2002, Mol. Cell Biol. 22, issue 16 and Xu et al., 1998, Science 279, 1922-1925, respectively.

The present invention further concerns special uses of cells and tissues from the above transgenic mammals in which the gene encoding the cofactor has been disrupted. Preferably, said cells and tissues are selected from the group consisting in adipocytes, hepatocytes, skeletal muscle cells, pancreatic beta cells, and related tissues. In preferred embodiment, said cells and tissues are derived from TIF2 -/- or from SRC-1 -/- null mice using standard methods of the art. More specifically, said cells and tissues are used to identify compounds that activate transcription through a PPAR-gamma /SRC pathway rather than through a PPAR-gamma /TIF2 pathway (or vice-versa).

Thus, the present invention further concerns a method of screening for compounds that activate transcription through a PPAR-gamma /SRC pathway rather than through a PPAR-gamma /TIF2 pathway, the method comprising :
(i) contacting :
   - at least one cell or tissue type that express PPAR nuclear receptors and that is derived from TIF-2 -/- transgenic mammal, and
   - at least one compound to be tested ,
   and
(ii) contacting :
   - at least one cell or tissue type that express PPAR nuclear receptors and that is of wildtype phenotype (i.e. no gene disrupted), and
   - the same compound to be tested as in (i),
(iii) comparing the effect of said compound to be tested on said cell or tissue in test (i) and in test (ii).

Alternatively, the present invention concerns a method of screening for compounds that activate transcription through a PPAR-gamma /TIF-2 pathway rather than through a PPAR-gamma /SRC-1 pathway, the method comprising :
(i) contacting :
   - at least one cell or tissue type that express PPAR nuclear receptors and that is derived from SRC-1 -/- transgenic mammal, and
   - at least one compound to be tested,
   and
(ii) contacting :
   - at least one cell or tissue type that express PPAR nuclear receptors and that is of wildtype phenotype (i.e. no gene disrupted), and
   - the same compound to be tested as in (i),
(iii) comparing the effect of said compound to be tested on said cell or tissue in test (i) and in test (ii).

According to special embodiment, said mammal is mouse.

According to special embodiment, said cell or tissue are selected from the group consisting in adipocytes, hepatocytes, skeletal muscle cells, pancreatic beta cells, and related tissues.

According to special embodiment, effect of said compound to be tested on said cell or tissue is evaluated by measuring efficacy of adipocyte differentiation and/or glucose uptake. Such methods of evaluation are well known in the art and disclosed in many papers (see for example.....).

All publications and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practised otherwise than as specifically described. Accordingly, those skilled in the art will recognize, or able to ascertain using no more than routine experimentation, many equivalents to the specific embodiments of the invention described specifically herein. Such equivalents are intended to be encompassed in the scope of the following claims.

These and other embodiments are disclosed or are obvious from and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on Internet, e.g. under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be identified/located using, e.g., http://www.lycos.com. An overview of patent information in biotechnology and a survey of relevant sources of patent information useful for retrospective searching and for current awareness are given in Berks, TIBTECH 12 (1994), 352-364.

### FIGURE LEGENDS

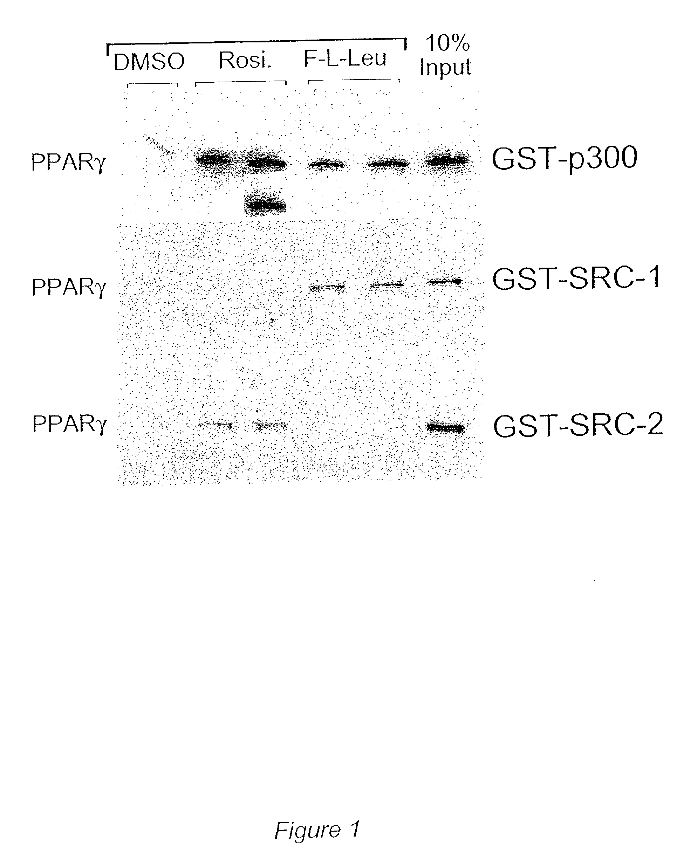

### Figure 1 : Interactions between PPAR-gamma and cofactors in vitro. GST pull-down assay of the PPAR-gamma ligand binding domain and various co-activators.

The purified His-tagPPARγ2DE203-477 protein was incubated with purified p300Nt-GST, TIF2-GST or SRC-1-GST proteins and glutathione-Q-Sepharose beads in presence of DMSO (0.1%), rosiglitazone (10⁻⁴ M) or FMOC-L-Leu (W002/00611) (10⁻³ M). The beads were then washed and the samples separated on SDS-PAGE. Blots were developed with anti-histidine antibodies.

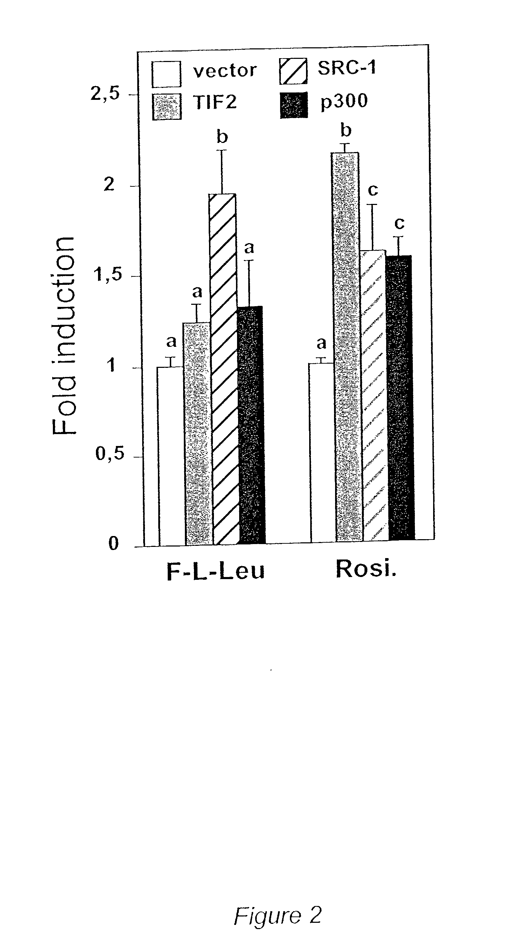

### Figure 2 : Interactions between PPAR-gamma and cofactors in cultured mammalian cells.

Mammalian two-hybrid interaction between PPAR-gamma and the various co-activators in RK13 cells co-transfected with an expression vector for BDGal4-hPPARγ DE, pGL3-(Gal4)5TKLuc reporter construct, pCMV-βGal, as a control of transfection efficiency, and different expression vectors for different cofactors. Cells were grown during 24 h in the presence or absence of the indicated compound. Luciferase activity has been normalized. Activation is expressed as fold induction relative to luciferase activity/β-galactosidase activity for transfection efficiency and harvesting efficiency using expression of cotransfected constitutive beta-galactosidase expression vector stimulated by FMOC-L-Leu (10⁻⁵M) or rosiglitazone (10⁻⁷M). Each point was performed in triplicate. Bars not sharing similar superscripts are statistically different from each other (P<0.05, Fisher's Post-hoc analysis of 2 x 2 ANOVA with ligands and constructs as factors).

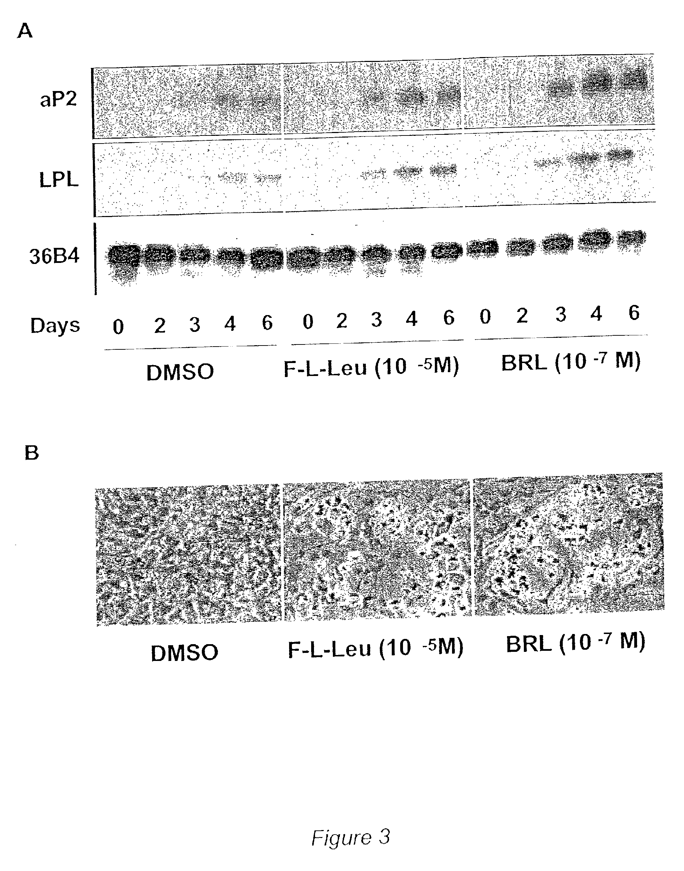

### Figure 3 : Ligands which favor recruitment of SRC-1 over TIF2 are less adipogenic in vitro.

A: Expression of adipocyte markers during differentiation of 3T3-L1 cells treated with different PPAR-gamma agonists. Confluent 3T3-L1 cells were incubated with 2 µM insulin, 1 µM dexamethasone, and 0.25 mM isobuthyl methyl xanthine for two days. Then, the cells were incubated in presence or DMSO (0.1 %), FMOC-L-Leu (10⁻⁵ M) or rosiglitazone (10⁻⁷ M) for 4 days. RNA was isolated from 3T3-L1 cells after different times of differentiation induction. Blots were hybridized with DNA probes that specifically bind to 36B4 (to control for RNA loading), lipoprotein lipase (LPL), or aP2.
B: Lipid accumulation in differentiating 3T3-L1 cells exposed to either rosiglitazone (10⁻⁷ M) or FMOC-L-Leu (10⁻⁵ M). Cells were stained with Oil Red O after 6 days of treatment.

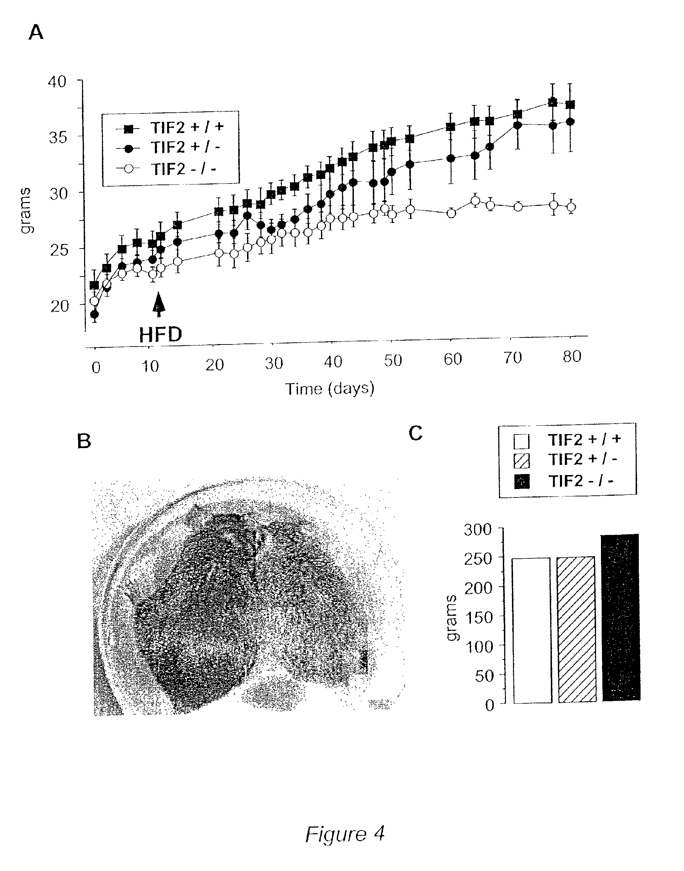

### Figure 4 : TIF2 -/- and +/- animals are resistant to diet induced obesity.

A: Growth curve of TIF2 wild-type (+/+), heterozygous (+/-) and knockout (-/-) mice aged 6 weeks old at day 0 (n=5). Note that high-fat diet was provided ad libitum from the 12^{th} day of life to enhance weight differences, but that statistical significance in body weight was already observed between TIF2 +/+ and -/- mice at that time (p < 0.05, ANOVA repeated measures).
B: Picture of representative wild-type (left) and knockout (right) TIF2 mice taken at the end of chronic high-fat feeding.
C: Cumulative food intake of TIF2 +/+, +/-, and -/- mice as measured every other day throughout of the treatment period. No difference was observed between the groups. Bars represent food intake per mouse, as estimated from overall food consumption (food given minus non-eaten food removed from cages).

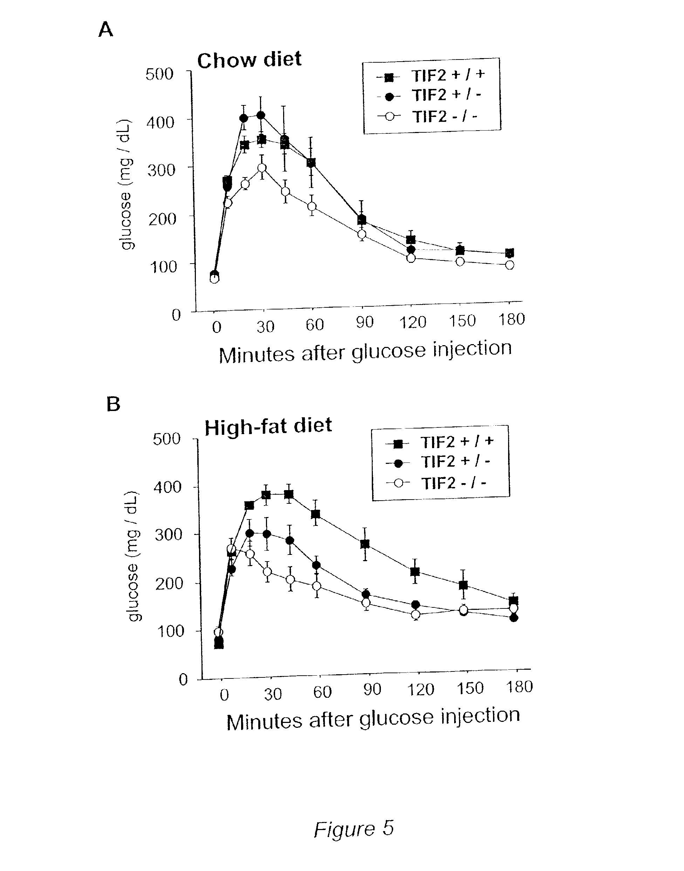

### Figure 5 : TIF2 -/- and +/- animals show an improved glucose sensitivity

A: Intraperitoneal glucose tolerance test performed in chow-fed TIF2 +/+, +/-, and -/- mice. Symbols represent means ± S.E.M. of 5 animals. TIF2 -/- mice had a lower glucose response compared to other genotypes (p < 0.05, ANOVA repeated measures).
B: Intraperitoneal glucose tolerance test performed in high-fat fed TIF2 +/+, +/-, and -/- mice. Symbols represent means ± S.E.M. of 5 animals. All glucose curves were significantly different from each other. (p < 0.05, ANOVA repeated measures).

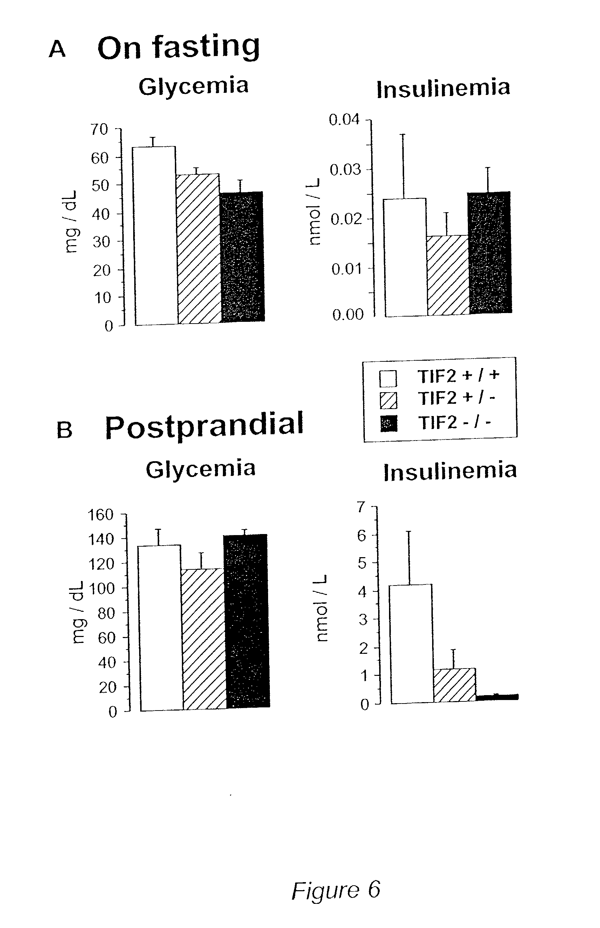

### Figure 6 : TIF2 -/- and +/- animals are insulin sensitized, when tested under postprandial conditions.

A: Glucose and insulin levels were measured after an overnight fast in TIF2 +/+, +/-, and -/- mice. Bars represent means ± S.E.M. of 5 animals. Plasma glucose levels were significantly lower in TIF2 knockout mice compared to wildtype littermates (p < 0.05). Note that no difference was observed in serum insulin levels, suggesting that less glucose was circulating in the blood despite similar insulin concentration.
B: Serum glucose and insulin levels determined in the postprandial state in TIF2 +/+. +/-, and -/- mice. Mice were allowed to eat for one hour after an overnight fast, and a blood sample was taken immediately after. Bars represent means ± S.E.M. of 5 animals tested. Note that glucose and insulin levels are higher compared to the fasted state. Lack of TIF2 results in a reduced need of insulin to maintain glycemia at a normal range, which is consistent with an improved insulin sensitivity.

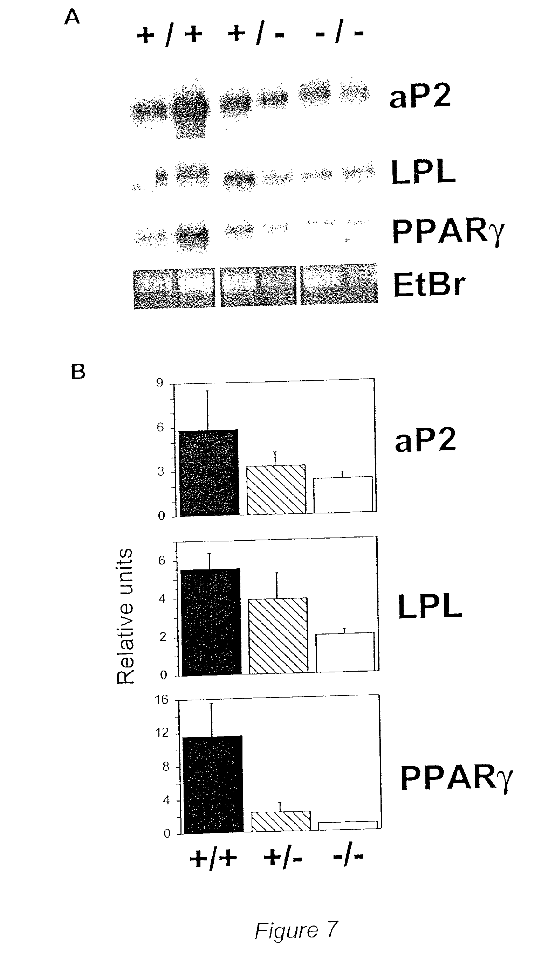

### Figure 7 : Decreased expression of PPAR-gamma target genes in TIF2 -/ - and +/- animals

A: Epididymal adipose tissue RNA was extracted and analyzed by Northern blotting for expression of aP2, LPL and PPAR-gamma. To control for loading, 28S mRNA levels were determined by staining the gel with ethidium bromide. Figure shows representative samples out of a group of 3-4 animals analyzed.
B: Relative units of density (arbitrary units) determined on the Northern blots described in 7A. Bars represent means ± S.E.M. of 3-4 animals tested. Significant differences in aP2 and LPL mRNA levels were observed between TIF2 wild-type and knockout mice, whereas all PPAR-gamma mRNA levels were different from each other (p < 0.05, one-way ANOVA).

### Figure 8 : TIF2 -/- annuals have lower serum triglycerides

Serum lipids were determined in the fasted state. Bars represent means ± S.E.M. of 5 animals tested. A slight but significant reduction in circulating triglycerides was observed in TIF2 knockout mice compared to wild-type littermates (p < 0.05, one-way ANOVA).

### Figure 9 : SRC-1 -/- and +/- animals show normal fat and glucose homeostasis.

A, B and C: Body weight and fasting glucose and insulin levels were determined in 12 weeks-old, chow-fed SCR-1 wild-type (+/+), heterozygous (+/-) and knockout (-/- ) mice. Bars represent means ± S. D. of 10 animals tested.
D: Intraperitoneal glucose tolerance test performed in SRC-1 +/+, +/-, and -/- mice. Symbols represent means ± S.D. of 10 animals. No difference was observed between groups (ANOVA repeated measures).

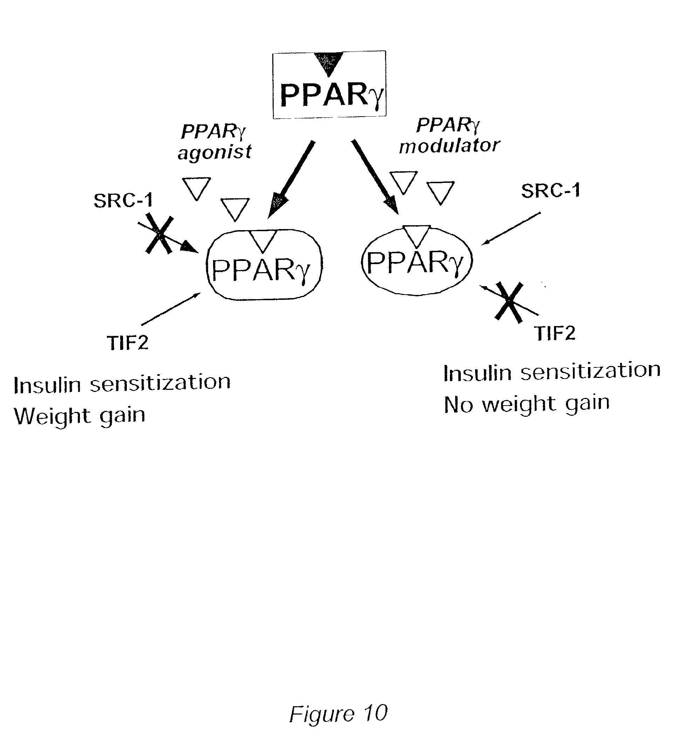

### Figure 10 : Schematic outline of the general principles underlying the PPAR-gamma modulator screening based on differential cofactor recruitment.

Representation of the impact of ligand-induced specific cofactor recruitment on PPAR-gamma activity and consequences on metabolism. The change in PPAR-gamma spatial configuration brought about by the binding of ligands, and indicated by the different shape of the receptors, triggers specific cofactor docking. This ultimately leads to transcription of genes that are differently expressed according to the class of ligand used, which ultimately has a major impact on energy metabolism.

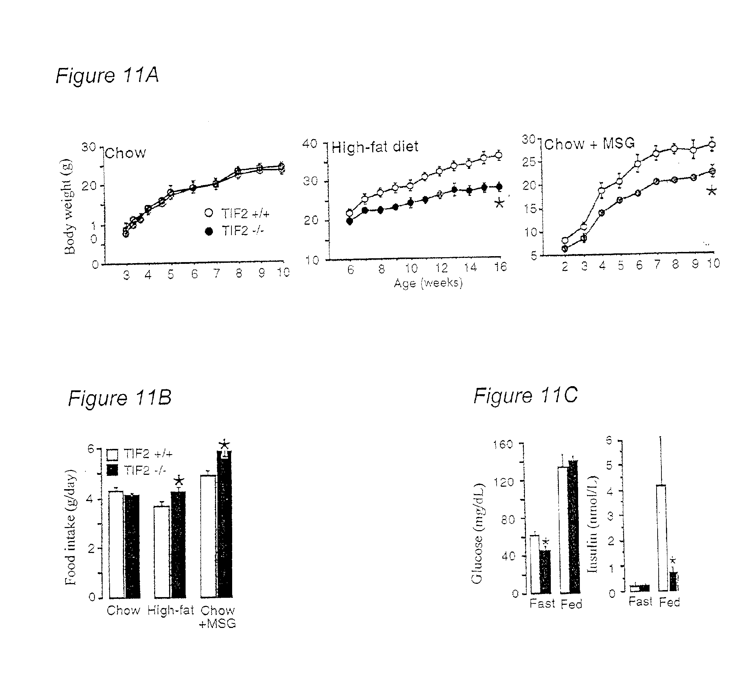

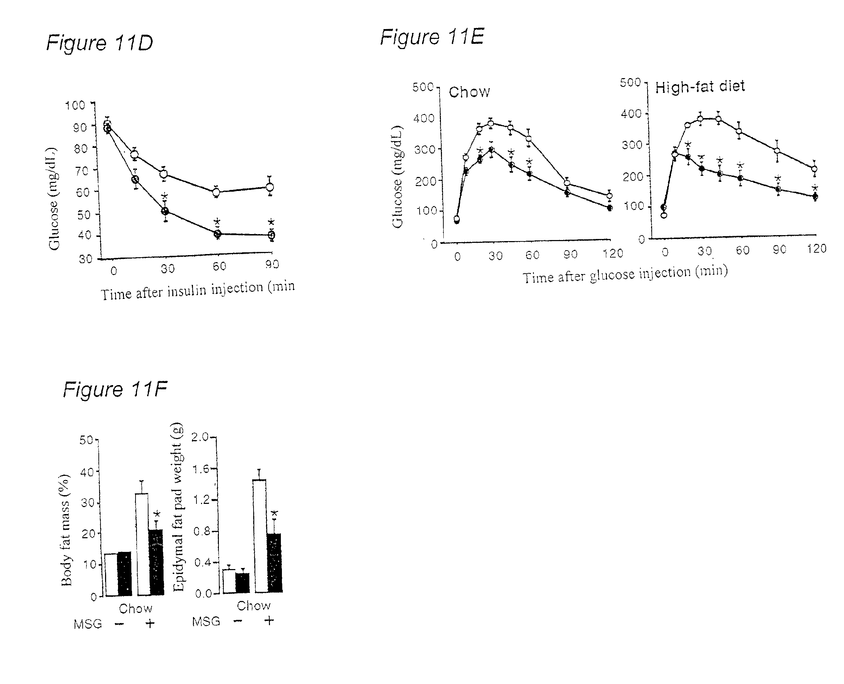

### Figure 11 Body weight regulation and glucose homeostasis in TIF2 knockout mice

[*, asterix] Indicates a significant difference in the phenotype analyzed between the +/+ and the -/- genotype. In each experiment, animals with an n = 5 were analyzed. Black symbols represent TIF2-/- mice, whereas white symbols represent their wild-type littermates.

A-B TIF2-/- mice have a lower body weight despite higher a food intake. Body weights were recorded weekly in TIF2+/+ and -/- animals either fed a regular chow diet, a high-fat diet, or in mice that have been treated neonataly with MSG (2 mg·kg⁻¹·day⁻¹, for 7 days) (A). Food intake per mouse was measured every second day for 15 days (B).

C-E TIF2-/- mice are insulin sensitized. (C) Plasma glucose and insulin levels after an overnight fast or 1 h following a standard meal. (D&E) Glucose levels after an acute injection of insulin (0.75 UI/kg) of after a glucose load (2 g/kg) performed as described (Rocchi et al., 2001, Mol Cell 8, 737-747) in chow fed (D&E) or high-fat fed mice (E).

F Lack of TIF2 protects against fat mass accumulation and obesity. Body fat mass was evaluated by DEXA scanning and determination of epididymal WAT weight in TIF2+/+ and -/- mice fed a chow diet with or without neonatal exposure to MSG (F).

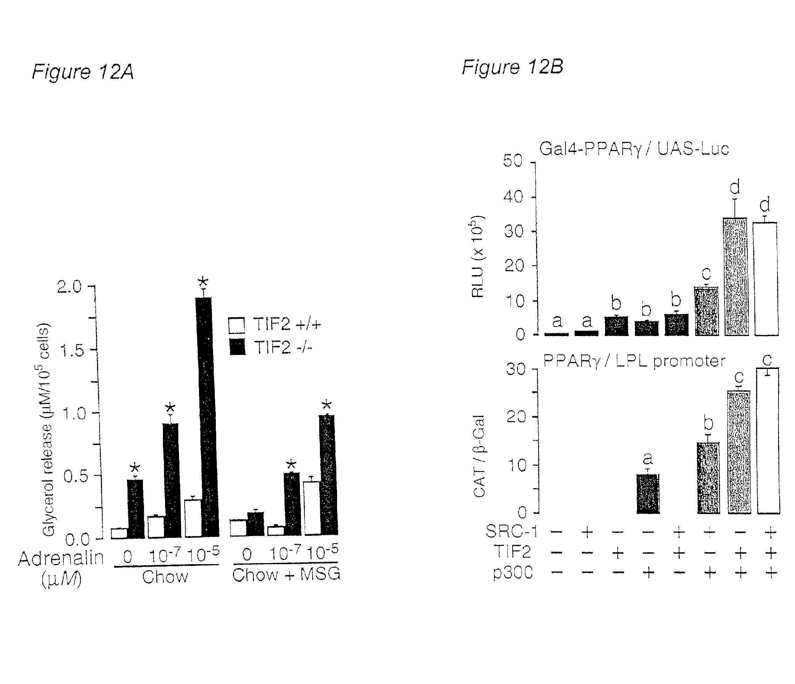

### Figure 12 Biology of TIF2-/- white adipocytes.

A Enhanced lipolysis in TIF2-/- adipocytes. (A) Isolated adipocytes from TIF2+/+ and -/- mice raised under normal conditions or after neonatal exposure to MSG were treated with 0, 10⁻⁷M and 10⁻⁵M adrenaline (Marette et al., 1991, Am J Physiol 261, E204-13) and the release of glycerol was analyzed 30 min later. * See Fig.1 for statistical significance.
B PPAR-gamma interaction with p300 is influenced by p160 coregulators. GST pull-down assays were performed with His-tagPPARgamma2 DE203-477 and increasing amounts (0.5, 1.5, 3 µg) of GST-p300, GST-SRC-1 or GST-TIF2 proteins (H) (Rocchi et al., 2001, supra). HEK 293T cells were transfected with pCMV-p300 (Gelman et al.,1999, J Biol Chem 274, 7681-7688), pcDNA-TIF2 ( Voegel et al, 1998, EMBO J 17, 507-519), pcDNA-SRC-1 ( Spencer, G. et al., 1997, Nature 389, 194-198) and either BDGa14-hPPAR-gamma DE (Oberfield et al., 1999, Proc Natl Acad Sci 96, 6102-6106) and the pGL3-(Gal4)5TKLuc reporter construct (Rocchi et al., 2001, supra) (upper panel), or pSG5-hPPARgamma2 encoding the full length receptor (Gelman et al., 1999, J Biol Chem 274, 7681-7688) and the LPL promoter-CAT reporter construct (Schoonjans et al., 1996, EMBO J. 15, 5336-5348) (lower panel). Cells were then grown for 24 h in the presence of rosiglitazone (10⁻⁷M). Luciferase activity is expressed as light units/beta-galactosidase activity. Each point was performed in triplicate. Bars not sharing similar superscripts are significantly different from each other.

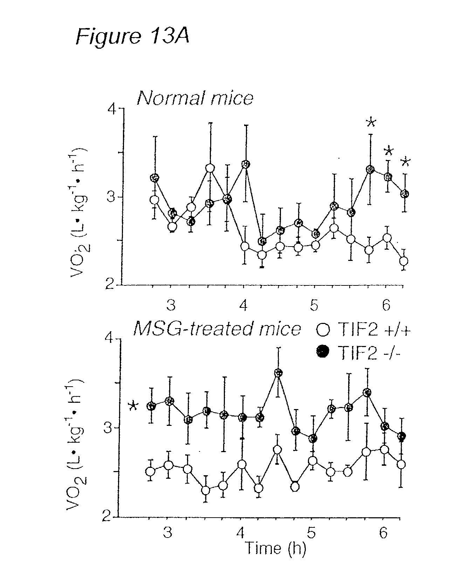

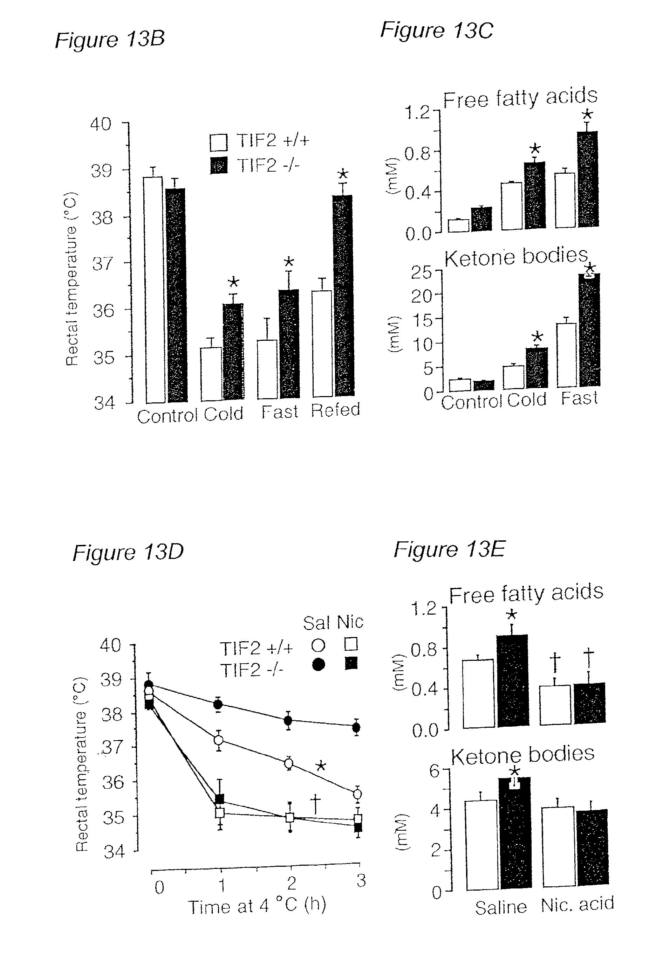

### Figure 13 BAT characteristics, oxygen consumption, and adaptive thermogenesis in TIF2-/- mice.

A TIF2 alters the blown adipocyte machinery (A) Oxygen consumption (VO₂) analyzed by indirect calorimetry in an open circuit in non-fasted TIF2+/+ and -/- mice raised under normal conditions (upper panel) or after neonatal exposure to MSG (lower panel).
B-E TIF2 knockout mice display higher adaptive thermogenesis. Rectal body temperature (B) and plasma levels of free fatty acids and ketone bodies (C) were evaluated in chow-fed TIF2+/+ and -/- mice either in a normal state, after 6 h of cold exposure, after an overnight fast, or 1 h following subsequent refeeding. Rectal body temperature (D) and plasma levels of free fatty acids and ketone bodies (E) in TIF2+/+ and -/- mice injected with either saline (Sal) or nicotinic acid (Nic; 150 mg/kg) and exposed to cold (4°C) one hour later. The reduced levels of free fatty acids in nicotinic acid-injected animals indicate the efficiency of the treatment.

* See Fig.11 for definition of statistical significance. † Indicates significant differences between saline and nicotinic acid-treated animals within the same genotype.

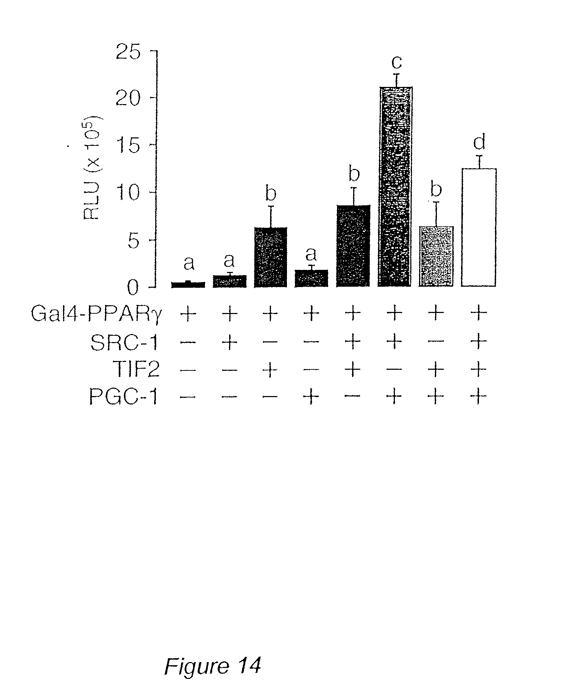

### Figure 14 Control of PGC-1/PPAR-gamma interactions by SRC-1 and TIF2.

TIF2 decreases the SRC-1-mediated interaction between PPARgamma and PGC-1. HEK 293T cells were transfected with expression vectors for BDGal4-hPPARgamma DE, the pGL3-(Gal4)5TKLuc reporter construct, and expression vectors for SRC-1, TIF2 and PGC-1. Cells were then grown for 24 h in the presence of rosiglitazone (10⁻ ⁷M). Luciferase activity is expressed as light units/beta-galactosidase activity. Each point was performed in triplicate.

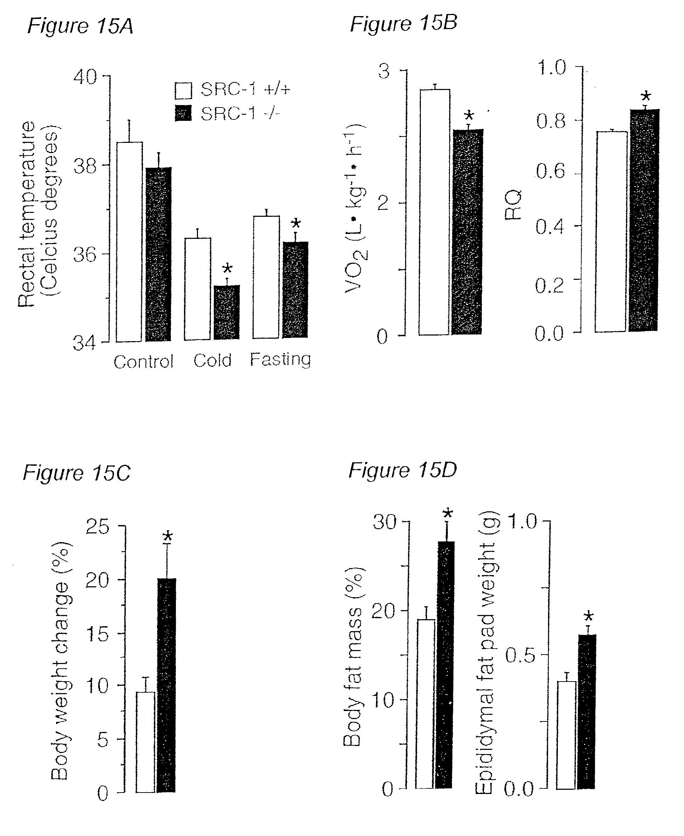

### Figure 15 Lack of SRC-1 impairs adaptive thermogenesis by BAT.

A-B SRC-1-/- mice have a reduced adaptive thermogenesis. Rectal body temperature was evaluated in high-fat fed SRC-1+/+ and -/- mice in a normal state (23°C), after cold (4°C) exposure for 6 hours or after an overnight fast (A). (B) Oxygen consumption (VO₂) and respiratory quotient (RQ) analyzed by indirect calorimetry in non-fasted animals.
C-D SRC-1-/- mice are prone to obesity. SRC-1+/+ and -/- mice were fed a high-fat diet. Body weight changes (C) and evaluation of fat mass accumulation (D) by DEXA scanning and determination of the epididymal WAT weight were recorded in these animals after 8 weeks of feeding. * See Fig.11 for definition of statistical significance.

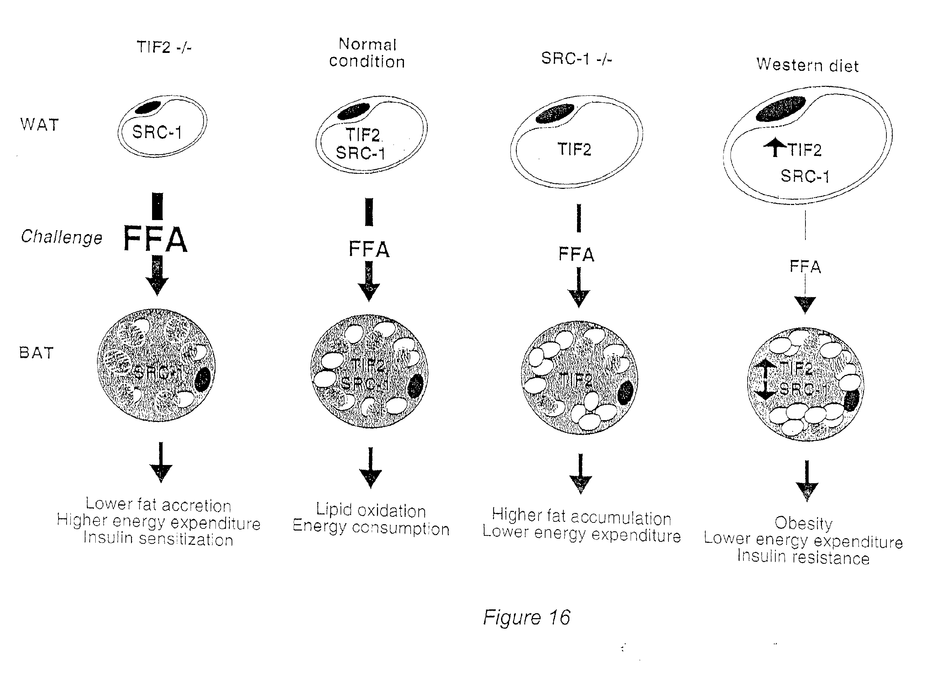

### Figure 16 Tissue-specific modulation of SRC-1 TIF2 in diet-induced obesity.

Role of p160 nuclear coregulators in energy metabolism. Scheme illustrating the hypothetical role of TIF2 and SRC-1 in obesity and insulin resistance. See text for details.

### METHODS

### PPAR-gamma ligands

- Rosiglitazone: Rosiglitazone is a high affinity PPAR-gamma ligand (Lehmann, et al., 1995, J. Biol. Chem. 270, 12953-12956) and is a member of the thiazolidinedione class of compounds. Rosiglitazone possesses the capacity to activate PPAR-gamma *in vitro* during transfection assays, to induce adipogenesis both *in vitro* and *in vivo* (Fajas et al., 1998, Curr. Opin. Cell Biol. 10, 165-173 ; Spiegelman, 1998, Diabetes 47, 507-514), and to improve insulin sensitivity in diabetic animals and humans (Glass et al., 1997, Curr. Opin. Cell Biol. 9, 222-232). The co-crystal structure of ligand binding domain of PPAR-gamma complexed to rosiglitazone is also known.
- L-tyrosine based ligands:Recently, a series of L-tyrosine based PPAR-gamma ligands was designed by replacing the thiazolidinedione ring with a carboxylic acid and by introducing an amine function on the adjacent carbon while keeping the parahydroxybenzyl sequence. An optimal PPAR-gamma activity was obtained when the amine on the alpha carbon of the L-tyrosine ligands was substituted with a benzoylphenyl function, leading to the development of N-(2-benzoylphenyl)-L-tyrosine derivatives (Cobb et al., 1998, J Med Chem 41, 5055-5069 ; Collins, et al., 1998, J Med Chem 41, 5037-5054 ; Henke et al., 1998, J Med Chem 41, 5020-5036). Rigidifying the benzoyl and phenyl moieties of this alpha-amino substituent through an additional phenyl-phenyl bond leads to compounds with good potencies. The prototypical compound of this group of ligands is represented by the Glaxo-Wellcome compound G1 262570.
- FMOC-L-Leu: FMOC-L-leucine (FMOC-L-Leu) structure lacks the parahydroybenzyl sequence present in both the thiazolidinediones and developed L-tyrosine-based PPARγ ligands, and was described as a new potent insulin-sensitizing compound with unique PPARγ-activating and -binding properties (WO 02/00611).

### Fluorescence Resonance Energy Transfer (FRET) :

A complete review of this method is presented in Siegel et al, (June 2000) Science STKE (38). The efficiency of FRET depends on the separation distance and the orientation of the donor and acceptor fluorescent moieties. FRET can be manifested as a reduction in the intensity of the fluorescence emission from the donor moiety, reduction in the lifetime of the excited state of the donor moiety, or emission of fluorescent light at the longer wavelengths (lower energies) characteristic of the acceptor moiety when the sample is excited at a wavelength that is optimal for the donor. Tables of spectral overlap integrals are readily available in Beriman, Energy transfer parameters of aromatic compounds, Academic Press, New York and London, 1973. Intensity of the fluorescent signal from the donor, the intensity of fluorescent signal from the acceptor, the ratio of the fluorescence amplitudes near the acceptor's emission maxima to the fluorescence amplitudes near the donor's emission maximum, or the excited state lifetime of the donor can be monitored. Changes in the amount of FRET can be determined as a function of the change in the ratio of the amount of fluorescence from the donor and acceptor moieties. Changes in the absolute amount of indicator, changes in excitation intensity, and changes in turbidity or other background absorbances in the sample at the excitation wavelength affect the intensities of fluorescence from both the donor and acceptor approximately in parallel. Therefore the ratio of the two emission intensities is a more robust and preferred measure of cleavage than either intensity alone. Protein fusions containing BFP and GFP linked together have been employed for example in protease assays by Mitra et al. (1996, Gene, 173, 13-17). Alternatively, the invention can be practiced with CARLA, a similar technique, which has been described in Krey et al, 1997, Molecular Endocrinology, 11, 779-91.

### Two-hybrid systems :

This technique utilizes hybrid proteins to detect protein-protein interactions by means of direct activation of reporter-gene expression. In essence, one of the two putative protein partners is genetically fused to the DNA-binding domain of a transcription factor and the other protein is fused to a transcriptional activation domain. A productive interaction between the two proteins of interest will bring the transcriptional activation domain of an adjacent reporter gene (usually LacZ or a nutritional marker) giving a screenable phenotype. The transcription can be activated through the use of two functional domains of a transcription factor : a domain that recognizes and binds to a specific site on the DNA and a domain that is necessary for activation, Keegan et al., 1986, Science, 231, 699-704 and US 5,667,973. Preferably, this system is performed in mammalian cells in the context of the invention.

### GST-pull down assays

- Production of proteins. The p300Nt-GST, SRC-1-GST and TIF2-GST fusion proteins were generated by cloning the N-terminal part of the cDNA coding for the p300 protein (amino acids 2 to 516), the region comprised between amino acids 568 and 780 of SRC-1 (SEQ ID NO: 03), and the domain between amino acids 618 and 826 of TIF2 (SEQ ID NO: 01), respectively, downstream of the glutathione-S-transferase (GST) protein in the pGex4-T1 vector (Pharmacia, Orsay, France). The fusion proteins were then expressed in E. coli and purified on a glutathione affinity matrix (Pharmacia). 6x-histidine-tagged human PPAR-gamma LBD (residues 203-477 of SEQ ID NO: 06) was overproduced in E. coli BE21 (DE3) using the pET15b as an expression plasmid (Novagen, Madison, WI) and purified by metal ion affinity chromatography on a Co²⁺-loaded HiTrap Chelating column (Pharmacia Biotech, Peapack, NJ) followed by gel filtration chromatography on a HiLoad Superdex 100 column (Pharmacia). The protein concentration was determined by the Bradford method (1976, Anal Biochem 72, 248-254).
- Pull-down experiments. The purified his-tagPPARγ DE proteins were incubated 1 hour at 22°C in pull-down buffer (phosphate-buffered saline 1x. Glycerol 10%, NP40 0,5%) with either GST, p300Nt-GST, SRC-1-GST, or TIF2-GST fusion proteins, glutathione-Q sepharose beads, and F-L-Leu (10⁻³M) or rosiglitazone (10⁻⁴M) when necessary. The beads were then washed 4 times with pull-down buffer and boiled in 4x-sample buffer. The samples were separated by 12% acrylamide SDS-PAGE and transferred to nitrocellulose membranes. Western blots were performed with antibodies directed against polyhistidine aminoacid sequences.

### Mammalian two-hybrid assays

RK-13 cells were obtained from ATCC (Rockville, MD). Cells were maintained in Dulbecco's modified Eagle's minimal essential medium (DMEM) supplemented with 10% fetal calf serum (FCS), L-glutamine, and antibiotics. Transfection studies with luciferase (luc.) reporter constructs were carried out as described previously (Oberfield et al., 1999, Proc Natl Acad Sci U S A 96, 6102-6106). The pGL3-(Gal4)5TKLuc reporter constructs are described elsewhere (Gelman et al., 1999, J. Biol. Chem. 274, 7681-7688).

The following expression vectors were used: pcDNA3-BDGal4-hPPARγ DE, encoding chimeric proteins composed of the Gal4 DNA binding domain fused to the hPPARγ ligand binding domain and pCMV-p300-CHA (Gelman et al., 1999, J. Biol. Chem. 274, 7681-7688) ; pSG5-TIF2 (Voegel et al, 1998, EMBO J. 17, 507-519); pcDNA-SRC-1 (Spencer at al., 1997, Nature 389, 194-198) and pCMV-βGal, as a control of transfection efficiency. Luciferase activity was taken as a direct evidence for PPARγ activity.

### Adipocyte differentiation

3T3-L1 cells (ATCC, Rockville, MD) were grown to confluence in medium A (Dulbecco's modified Eagle's Medium with 10% fetal calf serum, 100 units/ml penicillin, and 100µg/ml streptomycin). Confluent cell were incubated in medium A containing 2 µM insulin, 1 µM dexamethasone, and 0.25 mM isobuthyl methyl xanthine for two days. Then, the cells were incubated in medium A in presence or absence of PPARγ agonist for 4 days, changing the medium every 2 days. Adipogenesis was evaluated by analysis of the expression of adipocyte-specific markers and by staining of lipids with Oil Red O (Chawla & Lazar, 1994, Proc. Natl. Acad. Sci. USA 91, 1786-1790).

### RNA preparation and analysis

RNA was isolated from 3T3-L1 cells by the acid guanidinium thiocyanate/phenol/chloroform method (Chomczynski & Sacchi, 1987, Anal. Biochem. 162, 156-159). Northern blot analysis of total cellular RNA was performed as described (Auwerx et al., 1989, Mol. Cell. Biol. 9, 2298-2302). Lipoprotein lipase (LPL), aP2 and 36B4 were used as probes (Lefebvre et al., 1997, Arterioscler.Thromb.Vasc.Biol. 17, 1756-1764 ; Laborda, 1991, Nucl. Acids Res. 19, 3998 : Graves et al., 1992, Mol. Cell. Biol 12, 1202-1208).

### Animal experiments and glucose metabolism

All mice were maintained in a temperature-controlled (25 °C) facility with a strict 12 h light/dark cycle and were given free access to food and water. The standard rodent chow was obtained from Purina (diet # 5001; Ralston, Purina) and contains 23 % protein, 4.5 % fat, 6 % fiber, 8 % ash, and 56 % carbohydrate. The high-fat high-simple-carbohydrate diet contains 20.5 % protein, 35.8 % fat, 0.4 % fiber, 3.6 % ash, 3.1 % moisture and 36.8 % carbohydrates, primarily as disaccharides (diet # 1850; Bio-Serve, Frenchtown, NJ). Animals received FMOC-L-Leu or rosiglitazone by intraperitoneal injection.
C57B1/6J and db/db mice (8 per group) were obtained through the Janvier laboratories (Laval-Le Genest, France). We used female and male heterozygous mice, 10-12 weeks of age in which the SRC-1 (Xu et al., 1998, Science 279, 1922-1925) or TIF2 gene disruption was maintained on a hybrid genetic background of parental C57BL/6J and 129/SV mouse strains. Heterozygous mice were interbred to generate litters containing homozygous (-/-), heterozygous (+/-) and wild type (+/+) progeny.

Blood was collected in fasting mice from the retroorbital sinus plexus under chloroform anesthesia into tubes containing anticoagulant (1mM EDTA). Plasma was separated by low-speed centrifugation and kept at 4 °C until analysis (<2 days). Intraperitoneal glucose tolerance tests (IPGTT) were performed as described (Kaku et al., 1988, Diabetes 37, 707-713). Briefly, mice were fasted overnight (16h) and injected intraperitonealy (i.p.) with 25 % glucose in sterile saline (0.9 % NaCl) at a dose of 2 g glucose/kg body weight. Blood was subsequently collected from the tail for glucose quantification with the Maxi Kit Glucometer 4 (Bayer Diagnostic, Puteaux, France) prior to and at indicated times after injection. Blood for insulin measurement was collected under the conditions specified (fasting or non-fasting) from the retroorbital sinus plexus under chloroform anesthesia. Plasma was separated and insulin measured using a radio immunoassay kit (Cis bio international, Gif-sur-Yvette, France). Total cholesterol (TC), triglycerides (TG), HDL-cholesterol (HDL-C) and non HDL-cholesterol (non HDL-C) were determined by enzymatic assays adapted to microtiter plates using commercially available reagents (Boehringer-Mannheim, Germany). Plasma HDL content was determined after precipitation of apoB-containing lipoproteins with phosphotungtsic acid/Mg (Boehringer-Mannheim, Germany). Lipoprotein cholesterol profiles were obtained by fast protein liquid chromatography (FPLC) as described (Lefebvre et al., 1997, Arterioscler.Thromb.Vasc.Biol. 17, 1756-1764). This system allows for separation of the three major lipoprotein classes, VLDL, LDL and HDL. Cholesterol concentrations were determined in the eluted fractions. For the determination of the HDL particle composition, the HDL lipoprotein fraction (d = 1.063-1.21 g/ml) was isolated by sequential ultracentrifugation as described (Havel et al., 1955, J. Clin. Invest. 34, 1345-1353). The HDL fraction was then assayed for its protein (Lowryet al., 1951, J. Biol. Chem. 193, 265-275) and lipid (cholesterol, triglycerides and phospholipide) content. Results are expressed as mass percentage of HDL. Values correspond to one HDL preparation isolated from a pooled sample of 3 mice in each group.

### Statistical analysis

Values were reported as mean +/- standard deviation. Statistical differences were determined by either the Mann-Whitney U test or ANOVA analysis. A value of P<0.05 was considered as statistically significant.

### Example 1 : Distinct cofactor are recruited by different ligands; TIF2 is required for PPARγ transactivation.

The ligand-binding domain (DE domain) of PPARγ has been previously reported to interact with several cofactors, such as p300, SRC-1 and TIF2 (Glass & Rosenfeld, 2000, Genes Dev 14, 121-141), in a ligand-dependent fashion. Hence the purified PPARγ DE protein represents a tool to study PPARγ ligand binding properties in view of its ability to recruit cofactors upon ligand binding. Compared to the solvent (DMSO) control, both rosiglitazone and FMOC-L-Leu increased the interaction between the PPARγ DE and p300Nt-GST (Figure 1). FMOC-L-Leu, but not rosiglitazone, induced the formation of PPARγ DE/SRC-1-GST complexes. In contrast, an interaction between TIF2 and the PPARγ DE was detected only with rosiglitazone but not with FMOC-L-Leu. This result suggests that FMOC-L-Leu, upon binding to the PPARγ DE domain, induces a conformational change that has a higher affinity for association with p300 and SRC-1, than with TIF2. This condition is in contrast to the conformational change induced by rosiglitazone, which preferentially results in TIF2 recruitment to the PPARγ DE domain. These results support the hypothesis that PPARγ activation by different ligands induces the docking of specific cofactors.

The recruitment of cofactors by different PPARγ ligands was further tested in a modified mammalian two-hybrid system, using on the one hand a fusion protein between the PPARγ DE domain and the DNA binding domain of the yeast transcription factor Gal4, and on the other hand expression vectors for TIF2, SRC-1, and p300. The interaction between these proteins was tested in RK13 cells, which were incubated with either FMOC-L-Leu or rosiglitazone. Whereas no significant increase in transactivation was observed in the presence of TIF2 or p300 upon stimulation with FMOC-L-Leu, a much more pronounced and significant induction was seen with SRC-1, suggesting a more efficient recruitment of this co-activator (Figure 2). On the other hand, rosiglitazone had a significantly stronger effect on transactivation in the presence of TIF2, relative to SCR-1 or p300. These data extend in an intact cell system the in vitro results obtained in the GST-pull down assays. Taken together, these experiments strongly suggest that F-L-Leu activates PPARγ mainly through the recruitment of SRC-1 as a cofactor, whereas rosiglitazone modulates PPARγ activity via a mechanism that at least involves TIF2.

### Example 2 : FMOC-L-leucine has less potent adipogenic activity in the 3T3-L1 differentiation assay relative to classical PPARγ ligands.

Next the ability of FMOC-L-Leu and rosiglitazone to stimulate adipocyte differentiation of murine pre-adipocyte 3T3-L1 cells was next analyzed. Adipogenesis was monitored by analysis of lipoprotein lipase (LPL) and aP2 mRNA levels as markers of adipocyte differentiation and by studying morphological changes associated with the differentiation process. FMOC-L-Leu at the concentration of 10⁻⁵ M significantly stimulated both LPL and aP2 mRNA levels to an extent close to that seen in cells incubated with rosiglitazone at the concentration of 10⁻⁷ M (Figure 3A). Furthermore, staining of 3T3-L1 cells with Oil Red O, as a marker for neutral lipid accumulation, was performed after 6 day incubation with either DMSO, or the PPARγ ligands, FMOC-L-Leu or rosiglitazone, at the concentrations specified above (Figure 3B). The two drugs were capable of inducing neutral lipid accumulation, albeit with a different potency. FMOC-L-Leu hence was in fact much less adipogenic then rosiglitazone in 3T3-L1 cells, reflecting its relative affinity for the receptor.

WO 02/00611 demonstrated that FMOC-L-Leu improved insulin sensitivity in chow-fed and high fat-fed C57BL/6J, as well as diabetic db/db mice, yet it had lower adipogenic activity, then rosiglitazone. Thanks to the method according to the invention defined above, the Applicants have found that these biological effects are associated with differential cofactor recruitment and the Applicants next confirmed these results in vivo using various mice lines deficient in a particular cofactor, i.e. SRC-1 and TIF2 KO mice.

### Example 3 : TIF2 KO, but not SRC-1 KO mice, have abnormal PPARγ signaling providing further evidence that TIF2 is the preferred cofactor for PPARγ signaling.

Since the data above suggested that TIF2 was the preferred cofactor recruited to PPARγ by classical PPARγ agonists, such as the thiazolidinediones and the natural PPARγ ligands, the Applicants hypothesized that TIF2 KO animals should recapitulate some aspects of the PPARγ KO animals. Although PPARγ -/- animals are not viable, PPARγ +/- animals have a highly instructive phenotype. Consistent with the established role of PPARγ in adipocyte differentiation and fat accumulation, PPARγ +/- mice were protected against diet-induced obesity and were insulin sensitized (Kubotaet al., 1999, Mol. Cell 4. 597-609 ; Miles et al., 2000, J Clin Invest 105, 287-292 ; Barak et al., 1999, Mol. Cell 4, 585-595). To confirm a role of TIF2 in PPARγ signaling, in vivo, the Applicants analyzed metabolic function in detail in male TIF2 -/-, +/-, +/+ mice. Furthermore, the Applicants performed also a similar metabolic analysis in mice mutant for SRC-1, another member of the SRC/p160 cofactor family.

Already on a normal chow diet, TIF2 -/- animals weighed significantly less then the TIF2 +/- or +/+ mice. On a high fat / high sucrose diet, animals with TIF2 mutations were resistant to diet-induced obesity (Figure 4). This resistance to diet-induced obesity was most pronounced in TIF2 -/- animals and more moderate in the TIF2 +/- animals, suggesting that a gene dosage effect existed. Interestingly, although there was less weight gain in the TIF2 -/- mice, these mice had an equivalent and even slightly higher food intake, excluding differences in caloric intake as a potential explantion for the differences in weight. This resistance to diet-induced obesity is very similar to that observed in PPARγ +/- animals (Kubota et al., 1999, Mol. Cell 4, 597-609 ; Miles et al., 2000, J Clin Invest 105, 287-292).

In order to analyze whether glucose homeostasis in TIF2 KO animals was abnormal, the Applicants performed intraperitoneal glucose tolerance tests (IPGTT) in animals of the three different genotypes both on a chow diet as well as on a high fat /- high sucrose diet (Figure 5). On a chow diet the area under the glucose curve (AUC) during IPGTT was significantly lower in the TIF2 -/- animals; whereas the TIF2 +/- animals were indistinguishable from the wild-type animals. This is indicative of an improved glucose homeostasis in the TIF2 -/- animals. The differences in the AUC during IPGTT were more pronounced in animals on a high fat / high sucrose diet. On a high fat / high sucrose diet, a reverse relationship between TIF2 gene dosage and improvement in AUC was evident, confirming and further extending the data obtained on the chow diet. This improved glucose homeostasis was very similar to the effects observed in the PPARγ +/- animals. In another experiment, the Applicants tested insulin sensitivity in TIF2 KO animals, by measuring at the same moment serum glucose and insulin levels (Figure 6). Under fasting conditions, TIF2 -/- animals had significantly lower fasting plasma glucose levels, whereas no differences in insulin levels were detected (Figure 6A). This could be attributed to the extremely low insulin levels in the fasting animals. Although postprandial glucose levels were significantly higher then under fasting conditions, no difference in glucose levels was detected between the different genotypes (Figure 6B). Interestingly, insulin levels required, to maintain glucose levels postprandially were significantly lower in the TIF2 -/- and +/- animals. The decrease in insulin levels correlated with TIF2 gene dosage. These highly significant data point to the importance of TIF2 in glucose homeostasis and again mirror the results seen in the PPAR-gamma +/- animals.

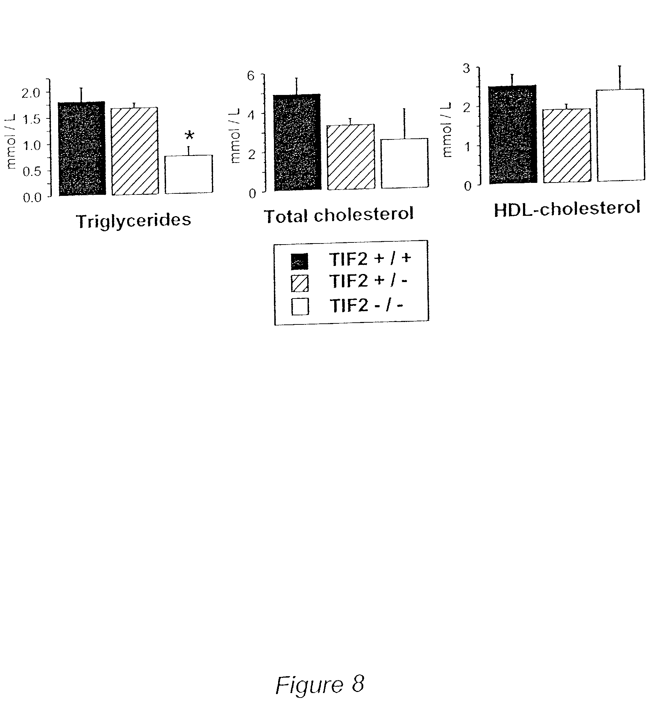

Further evidence showing that the TIF2 animals had a defect in the PPAR-gamma signaling was obtained through the analysis of the expression of PPAR-gamma target genes (Figure 7). As predicted the Applicants noted a gene-dose dependent decrease in the expression of two PPARγ target genes, such as lipoprotein lipase and the adipocyte-specific fatty acid binding protein aP2. Furthermore, TIF2 KO animals had significantly decreased fasting triglyceride levels, yet normal total and HDL cholesterol levels (Figure 8). Decreased serum triglyceride levels reflect often a decrease in adipose tissue mass, as present in the TIF2 KO animals, and suggests that TIF2 not only is involved in the regulation of glucose homeostasis, but also on lipid metabolism.

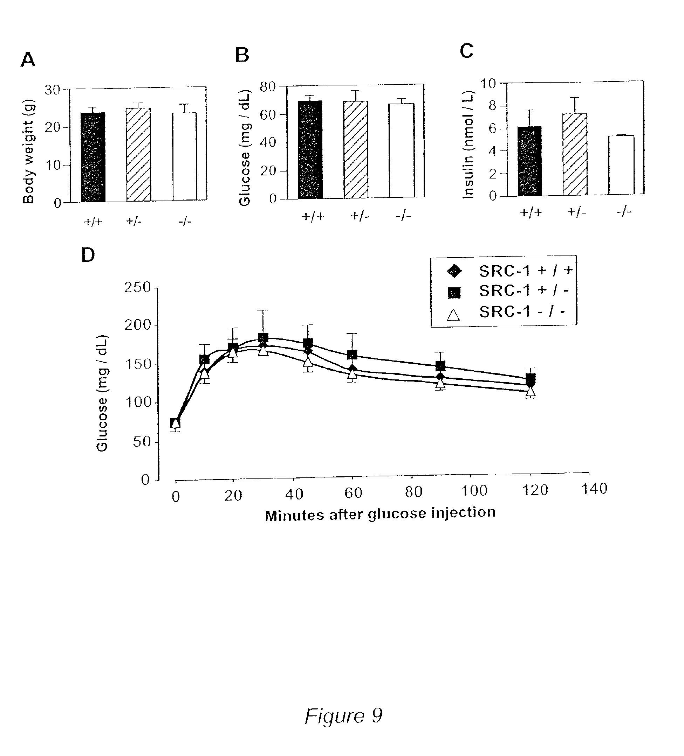

The phenotype seen in SRC-1 -/- mice was quite different. As summarized in Figure 9, SRC-1 mutation was not associated with a protection against obesity, since no differences in body weight were detected between SRC-1 -/-, +/-, and +/+ animals. Furthermore, SRC-1 -/- animals did not have abnormal glucose homeostasis and were not insulin sensitized. Hence the protection against excessive fat accumulation and obesity seems to be specific for the TIF2 KO animals and was not seen in the animals in which SRC-1, was mutated. In combination the phenotype observed in the TIF2 and SRC-1 KO animals provide compelling evidence suggesting that PPAR-gamma signaling is diminished in a TIF2 dose-dependent fashion and underscores that TIF2 (and not SRC-1) is the preferred p160 family member involved in transducing the PPARγ signal. These data furthermore the notion that the differential cofactor recruitment lead to distinct biological functions and validate *in vivo* the interactions between PPAR-gamma and different cofactors of the p160 family seen *in vitro* as described above.

### Example 4 : Outline for a cofactor-dependent screening method to identify PPARγ

Current available screening methods to identify new PPAR-gamma ligands are often cumbersome and do not allow to distinct between respective agonists, modulators, and antagonists of PPAR-gamma (or other nuclear receptors) in one single assay. The *in vitro* and *in vivo* evidence described above suggests that the unique reliance of classical PPAR-gamma agonists on TIF2 as a eofactor, points to a way to develop a novel fast screening method to distinguish upfront between pure PPAR-gamma agonists and modulators. From our studies, it is clear that classical PPARγ agonists (such as the thiazolidinediones and the natural PPARγ agonists) use preferentially TIF2 as cofactor. In contrast, PPARγ modulators preferentially recruit other cofactors, such as SRC-1. A screening method devised to distinguish between the receptor configuration, which allow one of these cofactors, but not the other to be recruited might be highly predictive of future in vivo effects of PPARγ ligands and might result in a significant improvement relative to current screening efforts. The Applicants outline in Figure 10 such a screening method, which relies on differential recruitment of different members of the SRC / p160 family of cofactors. Several methods can be used to score for cofactor recruitment and they range from biochemical to cell-based protein-protein interaction assays.

Examples include but are not limited to GST-pull down assays, FRET assays, classical and modified yeast two-hybrid assays, mammalian two-hybrid assays and CARLA. From our in vitro and in vivo validation studies the Applicants predict that compounds which recruit TIF2, are PPARγ agonists that will both enhance the adipogenic and insulin sensitizing activities of PPARγ, whereas compounds which will recruit preferentially SRC-1 behave like PPARγ modulators that are less adipogenic, yet retain insulin sensitization activities.

### Conclusions :

More specifically, using an in vitro pharmacological approach; the Applicants show that recruitment of TIF2 to PPAR-gamma translates in to greater adipogenic activity in the 3T3-L1 differentiation assay, relative to the recruitment of SRC-1. The Applicants also provide *in vivo* evidence to support this observation. In fact, whereas SRC-1 KO animals have an almost normal metabolic function, TIF2 KO animals have a severely deficient PPARγ signaling system and that mirrors the abnormalities seen in the heterozygote PPARγ +/- mice. In fact. TIF2 KO animals are resistant to diet induced obesity, are insulin sensitized, and exhibit a markedly improved glucose homeostasis. Furthermore, the data presented provide the first *in vivo* validation of the rational underlying a cofactor-based screening method for PPARs. Finally, these observations point to the importance of TIF2 as a novel target for the therapy of obesity; type 2 diabetes, and associated metabolic disorders, such as atherosclerosis, hypertension, etc.. The fact that predictions of activity, based on the cofactor recruitment assay, are recapitulated in the specific cofactor knock-out animals *in vivo*, underscores the validity of this screening approach and testifies for the novelty of these findings. Since the effects of these cofactors are known, this method may allow to predict the capacity of the PPAR-gamma ligand to induce specific metabolic processes (Figure 10).

### Example 5 : Generation of TIF2 ablated (TIF2^{-/-}) mutant mice (see also, Figure 1 in Gehin et al, 2002, August, Mol. Cell Biol. 22, issue 16)

To disrupt mTIF2 (the mouse homolog of human TIF2, also known as GRIP1), the Applicant deleted the exon encoding the nuclear receptor interaction domain NID (Voegel et al., 1998. Embo J 17(2): 507-19), using the Cre-lox technology and a targeting vector in which three loxP sites flank that exon and the TK-neo cassette. Complete Cre-mediated excision of the NID sequence in the TIF2 allele L3 generated the allele L⁻ with a frame shift starting at Arg 375 (mutated in Ser) and extending up to a new stop codon at amino acid position 490 (Stallcup, M.R. sequence acession number U39060). Thus, the mutated TIF2 allele L- has the capacity to encode a 489 amino acid-long truncated protein, that lacks all TIF2 amino acids C-terminal of the first NID residue. Partial Cre-mediated excision of the TK-neo cassette yielded the conditional "floxed" TIF2 allele L2 .

Mouse TIF2 genomic clones were obtained by screening a 129/Sv embryonic stem (ES) cell DNA library, with a mouse TIF2 (GRIPI) cDNA probe. A targeting vector was generated from two overlapping genomic fragments containing the exon encoding the three NR boxes of the NID (nucleotides 1330 to 2597 (accession number U39060). The TKneo cassette from pHR56 (Metzger et al., 1995, Proc Natl Acad Sci U S A 92(15): 6991-5) was cloned into the Clal site, and a LoxP site followed by a EcoRI site was introduced at the Spel site by PCR-based site-directed mutagenesis. The BamHI-HindIII fragment of the targeting vector was electroporated into 129/SvPas H1 ES cells and G418 neomycin resistant clones were expanded (Lufkin et al., 1991, Cell 66(6): 1105-19).

ES cells containing a targeted TIF2 L3 allele were identified by Southern blot analysis of (i) BamHI-digested ES cell genomic DNA using a 5' (P5') external probe, and (ii) BamHI and Kpnl-digested ES cell genomic DNA using a 3' (P3') external probe and a "neo" probe. Targeted ES cells were injected into C57BL/6 blastocysts which were implanted in a pseudopregnant host of the same strain. Chimeric males were obtained that transmitted the mutation through crosses with C57BL/6 females, yielding heterozygous TIF2^{L3/+} mice (mice with a L3 allele and a WT(+) allele). TIF2^{L3/+} mice were bred with homozygous CMV-Cre transgenic mice (Dupé et al., 1997, Development 124(2): 399-410) to generate TIF2^{L-/+} mice (mice bearing one allele in which the exon encoding the NID and the selectable marker were deleted), as well as TIF2^{L2/+} mice (mice bearing one floxed allele in which the exon encoding the NID is flanked by LoxP sites). Inbreeding of TIF2^{L-/+} mice yielded TIF2^{L-/L-} (also designated as TIF2^{-/-} or TIF2KO) mice homozygous for the deletion of the exon encoding the NID, while inbreeding of TIF2^{L2/+} mice yielded homozygous conditional "floxed" TIF2^{L2/L2} mice.

Mouse genotyping on tail-biopsy DNA was performed by PCR using primers P1 (5'-CTGCACGGTGCCAGCAAAGC-3'), P2 (5'-GACCAGGGCTTGCTCAGAAC-3') and P3 (5'-CCCCTGGATTGTTCCAAAGG-3'). The size of P2-P3 fragment from wild type is 289 bp, that of the P1-P3 fragment from the TIF2^{L-} allele is 512 bp and that of the P2-P3 fragment from the TIF2^{L2} allele is 405 bp).

Chimeric males originating from two independently targeted L3 ES clones (identified by Southern blotting) transmitted the mutation through their germline. To perform the Cre recombinase-mediated excision of the NID, heterozygous TIF2^{+/L3} mice were mated with CMV-Cre mice (Dupé et al., 1997, Development 124(2): 399-410). Inbreeding of the heterozygous TIF2^{+/L-} offspring (identified by PCR) yielded TIF2^{L-/L-} mice. A total of 561 offspring were obtained at the weaning stage from heterozygous parents including 173 TIF2^{+/+} (31%), 288 TIF2^{+/L-} (51%), and 100 TIF2^{L-/L-} (18%) mice. The lower-than-expected percentage of TIF2^{L-/L-} mice was due to death during the first month of their life, as E18.5 fetuses and 5 day-old (P5) newborns were found in a Mendelian distribution. The absence of the NID in the putative mutated TIF2 protein was assessed by immunohistochemistry with an antibody recognizing an epitope within the WT mouse TIF2/GRIP1 residues 468 and 481 (Puustinen et al., 2001. Eur J Endocrinol 145(3): 323-33). The strong signal observed with this antiboby in WT testes was clearly absent in TIF2^{L-/L-} testes.

### Example 6 : SRC-1 and TIF2 control energy fluxes between white and brown adipose issues.

The Applicants have explored the effects of two members of the p160 family of cofactors on energy homeostasis. They have shown that TIF2-/- mice are protected against obesity and display an enhanced adaptive thermogenesis, whereas SRC-1-/- mice (Xu et al., 1998, Science 279, 1922-1925) are prone to obesity due to reduced energy expenditure. Similarly, they concluded that in white adipose tissue (WAT), the lack of TIF2 translates into a lower PPAR-gamma activity, whereas in brown adipose tissue (BAT), it facilitates the interaction between SRC-1 and PGC-1, which induces the thermogenic activity of PGC-1. Interestingly, the ratio of TIF2/SRC-1 in WAT is also increased by high fat feeding, contributing to the weight inducing effects of such diets. These results demonstrate that the ratio between TIF2 and SRC-1 modulates energy metabolism.

### 6.1 TIF2-/- mice are protected against obesity and insulin resistance

On a regular chow diet, TIF2+/+ and -/- mice gained similar amounts of weight (Fig. 11A). TIF2-/- animals were, however, protected against the induction of obesity either by high-fat feeding or by overfeeding after neonatal injection of monosodium glutamate (MSG) (Fig. 11A). The differences in body weight are unlikely to be caused by a decrease in food intake, as TIF2-/- animals consumed more food in the two obesity models (Fig. 11B). These studies suggested that TIF2 modifies energy balance, most likely through the enhancement of energy expenditure.

Since glucose homeostasis is often correlated with body weight regulation (Saltiel, 2001, Cell 104, 517-529). we explored glucose metabolism in TIF2-/- mice. TIF2-/- animals had a lower fasting glycemia than their wild-type littermates (Fig. 11C). One hour after the beginning of a meal following an overnight fast, insulin levels of TIF2-/- mice were significantly lower compared to wild-type animals despite similar glucose levels (Fig. 11C). This suggested that TIF2-/- mice require less insulin to maintain their glycemia. Further; the rate of glucose clearance induced by an insulin injection was higher in TIF2-/- than in TIF2+/+ mice fed a chow diet (Fig. 1ID). Chowfed TIF2-/- mice had also a lower excursion of glucose levels than TIF2+/+ after a glucose injection (Fig. 11E). As expected, TIF2+/+ animals fed a high-fat diet for 10 weeks cleared less glucose over time after glucose injection than chow-fed TIF2+/+ mice, whereas TIF2-/- mice were protected against the alterations in glucose homeostasis induced by the high-fat diet (Fig. 1E). Whole-body insulin sensitivity was hence enhanced in the absence of TIF2.

Since TIF2-/- mice were protected against obesity, we investigated the impact of the mutation on fat mass accumulation. No difference was observed in total body fat content between chow-fed TIF2+/+ and -/- mice as measured by dual energy X-ray absorptiometry (Fig. 11F). In contrast, after neonatal exposure to MSG, TIF2-/- mice had a significantly lower total body fat content than their wild-type counterparts (Fig. 11F). These differences were mirrored by a lower weight of the epididymal white adipose tissue (WAT) in TIF2-/- than in TIF2+/+ mice upon MSG treatment (Fig. 11F). The applicants have furthermore shown that in TIF2-/- mice, the size of the white adipocytes was dramatically reduced even under a regular chow diet, as assessed by both classical histology and scanning electron microscopy.

### 6.2 TIF2 affects white adipose tissue by modulating lipolysis and adipogenesis

The difference in both adipocyte size and fat pad mass can be the consequence of an increased lipolysis, decreased capacity for lipid accumulation or a combination of both. Therefore, the Applicants have next evaluated glycerol release in primary cultures of isolated adipocytes from TIF2+/+ and -/- mice which were either raised under normal conditions or had been treated with MSG as neonates. In adipocytes isolated from both the chow and the NISG/chow group, lipolysis was higher in TIF2-/- cells both under basal conditions and upon exposure to adrenaline (Fig. 12A). The absence of TIF2 therefore enhances lipolytic responsiveness to the sympathetic nervous system, an effect that is usually associated with smaller fat pads. Perilipins are anti-lipolytic lipid droplet binding proteins and their absence in mice has been associated with resistance to obesity. Consistent with an increased lipolysis, the Applicants have shown that white adipocytes from TIF2-/- mice had lower levels of perilipin A and B compared to those of TIF2+/+ animals. The expression of genes involved in fatty acid uptake, such as fatty acid binding protein (aP2), lipoprotein lipase (LPL) and PPAR-gamma were also lower in TIF2-/- mice.

The Applicants then evaluated the effect of ectopic expression of TIF2 and SRC-1 on adipocyte differentiation by using a retroviral infection approach. In the presence of the differentiation cocktail, 3T3-L1 cells infected with a retrovirus expressing TIF2 accumulated significantly more lipids compared to cells infected with an empty control retrovirus or with a SRC-1-expressing retrovirus. To evaluate whether this effect of TIF2 is mainly mediated by PPAR-gamma, they also used NIH-3T3 cells, which can not be differentiated into adipocytes, because they lack PPAR-gamma (Tontonoz et al., 1994, Cell, 79, 1147-1156). In these rosiglitazone-treated NIH-3T3 cells, neither overexpression of TIF2- nor SRC-1-induced adipogenesis, providing further evidence that TIF2 directly modulates the PPAR-gamma mediated differentiation pathway.

The affinity of three distinct coregulators, SRC-1, TIF2, and p300 for rosiglitazone-bound PPAR-gamma was then compared by GST pull-down assays using increasing amounts of the various GST-coregulator fusion proteins and a fixed amount of the PPAR-gamma LBD protein. Recruitment of TIF2 by the LBD of PPAR-gamma increased when higher amounts of TIF2 were added and the interaction had roughly the same strength than that seen between the PPAR-gamma LBD and p300. To further validate this hypothesis, the Applicants have cotransfected a Gal4-PPAR-gamma LBD chimera. a UAS-driven luciferase reporter, and expression vectors for TIF2, SRC-1 and p300 (Rocchi et al., 2001, Mol Cell 8, 737-747). TIF2 is more effective than SRC-1 to enhance the activity of Gal4-PPAR-gamma (Fig. 12B). In the presence of p300, the absence of TIF2, but not that of SRC-1, decreased the activity of Gal4-PPAR-gamma(Fig. 12B). Similar findings were observed in transfection assays using full length PPARgamma and the human LPL promoter instead of the artificial UAS-Gal4 system (Fig. 12B). These results suggest that in the absence of TIF2, but not in the absence of SRC-1, the p300/PPARgamma complex was less active. Together, these data illustrate that the indirect reduction of PPAR-gamma activity imposed by the absence of TIF2 contributes to the decreased adipose tissue mass with smaller adipocytes due to impaired adipogenesis and enhanced lipolysis.

### 6.3 A more active brown adipose tissue in TIF2-/- enhances adaptive thermogenesis

Adaptive thermogenesis is an important component of energy balance and a metabolic defense against obesity through the stimulation of shivering and non-shivering thermogenesis in skeletal muscle and brown adipose tissue (BAT), respectively (Himms-Hagen, 1984, *N Engl J Med*., 311, 1549-1558). BAT is the main thermogenic tissue in rodents where fatty acid oxidation generates heat through the stimulation of the uncoupling protein-1 (UCP-1) (Klaus et al., 1991, Int J Biochem 32, 780-791).

The lower body weight observed in TIF2-/- mice despite a higher food intake suggested that energy expenditure was up-regulated in conditions promoting obesity (Fig. 11A&B), and led the Applicants to evaluate the morphological and functional characteristics of BAT in the TIF2-/- mice. Under macroscopic inspection, the interscapular BAT of TIF2-/- mice looked darker, and had less WAT surrounding it than that of TIF2+/+ animals. Intracellular lipid accumulation was decreased in TIF2-/- brown adipocytes, explaining at least in part the darker aspect of the BAT. The TIF2-/- brown adipocytes seemed collapsed and less rounded upon scanning electron microscopy and contained enlarged mitochondria with more cristae and much smaller lipid droplets in transmission electron microscopy.

The Applicants have then performed indirect calorimetric measurements in TIF2- /- animals. In non-fasting conditions, oxygen consumption by TIF2-/- mice was similar to that seen in wild-type animals (Fig. 13A), but as the mice were fasted, because they had no access to food in the metabolic cages, a statistically significant increase in oxygen consumption was observed (after ∼ 6 hours; Fig. 13A top panel). MSG-treated TIF2-/- mice had a significantly higher oxygen consumption throughout the test (Fig. 13A), which was consistent with the fact that TIF2-/- mice had a lower body weight despite a higher caloric intake (Fig. 11A&B). Since oxygen consumption is determined by mitochondrial activity and fatty acid oxydation in BAT, we compared gene expression in BAT between TIF2+/+ and -/- mice. Steady state mRNA levels of UCP-1, PGC-1 and acetylCoA oxidase (AOX) were all higher in TIF2-/- mice, whereas PPAR-gamma expression seemed reduced and SRC-1 mRNA levels were not altered. In addition, plasma concentrations of thyroid hormones were higher in TIF2-/- mice (T3: 0.85± 0.45 vs 1.29±0.18 nM, *p*=0.03; T4: 40.1±1.8 vs 78.6±6.1 nM, *p*=0.0003, in TIF2+/+ vs -/- mice, respectively), suggesting that the physiological adaptations due to the lack of TIF2 also involves hormonal changes that may affect thermogenesis. These findings indicated that the absence of TIF2 resulted in a better thermogenic capacity by fatty acid oxidation and uncoupling of respiration.

To further validate this hypothesis, mice were placed under conditions in which adaptive thermogenesis was challenged. Whereas no difference in rectal body temperature was observed under normal room temperature conditions (23°C, control), TIF2-/- mice had a higher body temperature than TIF2+/+ animals when exposed to 4°C overnight (Fig. 13B). In addition, overnight fasting, which generally decreases energy expenditure, was also accompanied by a higher rectal body temperature in TIF2-/- mice (Fig 13B). Also upon refeeding, diet-induced thermogenesis was more pronounced in TIF2-/- mice (Fig. 13B). In line with the higher lipolytic activity in white adipocytes lacking TIF2 (Fig. 12A), plasma free fatty acid levels were higher in TIF2-/- mice upon cold exposure and fasting (Fig. 13C). Moreover, circulating ketone bodies were higher in TIF2-/- mice (Fig. 13C), suggesting an enhanced fatty acid oxidation activity contributing to thermogenesis.

The Applicants have then established the contribution of fatty acids released from WAT as energy substrate for heat production in BAT. For that, mice were injected with nicotinic acid, an anti-lipolytic agent (Larsen and Illingworth, 1993, Curr Opin Lipidol 4, 34-40), one hour before being placed at 4°C. In these conditions, the characteristic elevation of circulating levels of free fatty acids was blunted (Fig. 13E), and differences in rectal temperature and circulating ketone bodies were no longer observed between TIF2+/+ and -/- mice (Fig. 13D). These data demonstrate that the increased lipolytic capacity in WAT of TIF2-/- mice contributes to BAT thermogenesis, and underscore that these two tissues are metabolically linked.

### 6.4 TIF2 and SRC-1 modulate PGC-1 transactivation properties

The Applicants have then compared the ability of SRC-1 and TIF2 to stabilize PGC- 1 binding to PPAR-gamma by co-immuno-precipitations of coregulator complexes from rosiglitazone-treated cells transfected with PPAR-gamma and various coregulators. The Applicants have observed that PPAR-gamma and PGC-1 physically interacted in the presence of SRC-1 and that TIF2 dose-dependently attenuated this interaction suggesting that it competes with SRC-1 for the formation of PGC-1/PPAR-gamma complexes. This hypothesis was further tested by transient transfection assays using a UAS-driven luciferase reporter and a Gal4-PPAR-gamma LBD fusion protein (Fig. 14). TIF2 is a better stimulator of PPAR-gamma compared to SRC-1 in the presence of rosiglitazone. PGC-1 only slightly increased PPAR-gamma activity by itself. SRC-1, but not TIF2, strongly enhanced PGC-1-mediated PPAR-gamma transactivation. Moreover, when compared to the condition in which the three coregulators were present (last bar), the absence of SRC-1 decreased, whereas the lack of TIF2 rather increased PPAR-gamma activity. These results confirmed that TIF2 and SRC-1 compete for interaction with PGC-1.

### 6.5 SRC-1-/- mice have a reduced energy expenditure and are prone to obesity

These observations indicate that the formation of a more active SRC-1/PGC-1 complex is favored in the absence of TIF2, which could explain, at least in part, why TIF2-/- mice have an enhanced energy expenditure. The absence of SRC-1 should therefore trigger the formation of a less active TIF2/PGC-1 complex. This hypothesis was tested by analyzing BAT morphology and function in SRC-1-/- mice (Xu et at., 1998, Science 279. 1922-5) fed a high-fat diet for 8 weeks. BAT of SRC-1-/- mice was characterized by the infiltration of white adipocyte streaks and contained significantly more intracellular lipids.

Next SRC-1+/+ and -/- mice were exposed to cold (4°C) for 6 hours or fasted overnight. The rectal body temperature of SRC-1-/- mice was significantly lower upon cold exposure and fasting than the temperature measured for SRC-1+/+ animals (Fig. 15A). These differences were associated with a lower concentration of circulating ketone bodies in SRC-1 -/- mice, suggesting a reduced fatty acid oxidation. Consistent with this, SRC-1-/- mice consumed significantly less oxygen as measured by in indirect calorimetry over a test period of 6 h (Fig. 15B). In addition, SRC-1-/- animals had a higher RQ (CO₂ formed/O₂ used) than their wild-type littermates during the same period (Fig. 15B), indicative of an attenuated fatty acid oxidation relative to that of glucose.

Chronic high-fat feeding in mice is known to increase energy expenditure by approximately 40% due to an attempt by the mouse to reduce body weight gain (Richard et al., 1988, Can J Physiol Pharmacol 66, 1297-302). To evaluate whether the diminished adaptive thermogenesis in mice lacking SRC-1 confers susceptibility to obesity, the Applicants have compared fat mass accumulation in SRC-1+/+ and -/- animals fed a high-fat diet for 8 weeks. SRC-1-/- mice gained 10% more weight during this period (Fig. 15C), which was mainly due to fat accumulation in WAT (Fig. 15D), as assessed by both DEXA scanning (+46% vs SRC-1+/+ mice) and weight determination of the epididymal white adipose depot (+41% vs SRC-1+/+ mice). Consistent with these observations, the mRNA levels of UCP-1. PGC-1 and AOX were all reduced in the BAT of SRC-1-/- mice compared to those of SRC-1+/+ animals, indicating that the thermogenic machinery in BAT was down-regulated in the absence of SRC-1. It is likely that the lower PGC-1 levels, along with the less stable and less active PGC-1/TIF2 transactivation complex imposed by the absence of SRC-1, contributed to the decreased thermogenic potential of BAT in SRC-1-/- mice. In sum, these findings demonstrate that mice lacking SRC-1 are prone to obesity as a result of their reduced capacity to increase their energy expenditure and fatty acid oxidation in response to high-fat feeding.

### 6.6 TIF2 and SRC-1 are inversely modulated upon high-fat feeding

Chronic ingestion of high-fat diets leads to altered energy balance, resulting in obesity and insulin resistance. The Applicants have therefore tested the effects of a high-fat diet for four months on the expression of TIF2 and SRC-1 in C57BL/6J mice. In WAT, TIF2 levels were significantly increased upon high-fat feeding, whereas SRC-1 expression remained unchanged. High-fat feeding robustly induced TIF2 expression in BAT, whereas there was a tendency for SRC-1 mRNA and protein to be reduced. These results suggest that high-fat diets modulate the ratio of the different p160 cofactors and imply that such changes could contribute to the alterations in energy balance occurring upon high-fat feeding leading ultimately to obesity and insulin resistance (Fig. 16).

### 6.7 Conclusions

The Applicants have demonstrated that cofactors of the p160 family control energy metabolism by affecting both WAT and BAT homeostasis. Based on these data, they formulated the hypothesis summarized in Fig. 16. In wild-type animals, lipolysis in WAT occurs upon challenge, such as cold exposure or fasting. The free fatty acids released into the circulation are taken up by thermogenic tissues such as the BAT, where they serve as fuel for energy expenditure and adaptive thermogenesis. In the absence of TIF2, lipolysis in WAT is enhanced, releasing more substrates for fatty acid oxidation in the hypertrophic mitochondria of the BAT. This stimulated energy expenditure protects from excessive fat accumulation, and keeps insulin sensitivity high. In contrast, the lack of SRC-1 reduces oxygen consumption and adaptive thermogenesis, which renders mice prone to obesity upon high-fat feeding.

These effects can be explained largely by the fact that TIF2 is the preferred p160 partner of PPAR-gamma activity in WAT and that the absence of TIF2 leads to decreased PPAR-gamma activity. The lack of TIF2 in BAT attenuates its competition with SRC-1 for PGC-1 and promotes the formation of stable and more active SRC-1/PGC-1 complexes that facilitate the transcriptional program of adaptive thermogenesis. In contrast, the absence of SRC-1 triggers the formation of a less active TIF2/PGC-1 complex, which reduces energy expenditure.

Thus, the method of the present invention allows alteration of the composition of cofactor complexes, and changes in the ratio of TIF2 and SRC-1, thereby modifying the transcriptional control of adipogenesis and thermogenesis regulation.

The observation that expression of SRC-1 and TIF2 is altered by high-fat diets is highly relevant. In fact, in conditions of high energy intake, such as chronic ingestion of a high-fat diet, TIF2 is increased in WAT. This enhances adipocyte differentiation and attenuates lipolysis, resulting in fat accretion in WAT. In BAT, the decreased ratio between SRC-1 and TIF2 will diminish the thermogenic potential. In such a setting, energy expenditure cannot compensate for the higher energy intake, promoting the development of obesity and insulin resistance (Fig. 16).

## Claims

1. A method of screening for compounds that modulate the interaction of at least one nuclear receptor with all or part of at least one nuclear receptor cofactor of the p160 family, the method comprising:
(i) incubating a reaction mixture comprising :
- at least one nuclear receptor, or fragments thereof,
- at least one potential binding modulator and,
- at least one nuclear receptor cofactor of the p160 family, fragments thereof, equivalents or chimeric proteins thereof, and
(ii) determining whether the binding to said nuclear receptor of said nuclear receptor cofactor, of said fragment or of said chimeric protein, is increased or decreased in comparison to an assay which lacks the potential binding modulator.

2. The method of claim 1, wherein said nuclear receptor cofactor of the p160 family is selected in the group consisting in TIF-2, SRC-1 and SRC-3.

3. A method of screening for compounds that modulate the interaction of at least one nuclear receptor with all or part of at least one nuclear receptor cofactor, the method comprising:
(i) incubating a reaction mixture comprising :
- at least one nuclear receptor, or fragments thereof,
- at least one potential binding modulator and,
- nuclear receptor cofactor TIF2, fragments thereof or chimeric proteins thereof, and
(ii) determining whether the binding to said nuclear receptor of said nuclear receptor cofactor, of said fragment or of said chimeric protein, is increased or decreased in comparison to an assay which lacks the potential binding modulator.

4. The method of claim 3, wherein said method is comprising:
(i) incubating a reaction mixture comprising :
- at least one nuclear receptor, or fragments thereof,
- at least one potential binding modulator and,
- nuclear receptor cofactor TIF2, fragments thereof or chimeric proteins thereof thereof,
and
(ii) incubating :
(a) a reaction mixture comprising :
- the same nuclear receptor, or fragments thereof, as in step (i)
- the same potential binding modulator as in step (i) and,
- nuclear receptor cofactor SRC1, fragments thereof or chimeric proteins thereof thereof,
and /or
(b) a reaction mixture comprising :
- the same nuclear receptor, or fragments thereof, as in steps (i)
- the same potential binding modulator as in step (i) and,
- nuclear receptor cofactor PGC-1, fragments thereof or chimeric proteins thereof,
and
(iii) determining whether the binding to said nuclear receptor of said nuclear receptor cofactors, of said fragments or of said chimeric proteins, is increased or decreased in comparison to assays which lack the potential binding modulator.

5. The method of claims 1 -4, wherein the reaction mixture of step (i) comprises a fragment of the nuclear receptor, said fragment including the C-terminal domain of said receptor.

6. The method of any of claims 1-5, wherein it is a screening method for compounds that inhibit the interaction of said nuclear receptor with said nuclear receptor cofactor TIF2.

7. The method of any of claims 1 -5, wherein it is a screening method for compounds that enhance the interaction of said nuclear receptor with said nuclear receptor cofactor TIF2.

8. The method of any of claims 1-7, wherein it is a screening method for compounds that enhance the interaction of said nuclear receptor with said nuclear receptor cofactor SRC-1.

9. The method of any of claims 1-8, wherein it is a screening method for compounds that enhance the interaction of said nuclear receptor with said nuclear receptor cofactor PGC-1.

10. The method of any of claims 1-9, wherein it comprises a further step consisting in assessing the effect of the modulation of the binding of said nuclear receptor cofactor(s) to said nuclear receptor on the transcriptional regulation activity of said nuclear receptor.

11. The method of any of claims 1-10, wherein it comprises a further step consisting in determining the cell and/or tissue specificity :
- of the modulation of the binding of said nuclear receptor cofactor to said nuclear receptor as determined in claims 1-9 , and/or
- of the effect of said modulation on the transcriptional regulation activity of said nuclear receptor as determined in claim 10.

12. The method of any of claims 1-11, wherein said nuclear receptor is the PPAR-gamma receptor.

13. A method for identifying a compound that modulates at least one nuclear receptor function comprising a screening method of any of claims 10-12.

14. A method for identifying a compound that modulates the PPAR-gamma function comprising a screening method of any of claims 10-12.

15. Compounds that modulate the interaction of at least one nuclear receptor at least with nuclear receptor cofactor TIF-2, wherein it is identified by a method of any of claims 1-14.

16. The compound of claim 15, wherein it is a PPAR-gamma receptor modulator.

17. The compound of claims 15 or 16, wherein said modulator is selected in the group consisting in a nuclear receptor agonists and nuclear receptor partial agonists.

18. A composition comprising at least one compound of any of claims 15-17 and a pharmaceutically acceptable carrier.

19. A method for treating and/or preventing diseases or pathologic conditions associated with cell types that express PPAR receptors comprising administering to the patient at least one compound of any of claims 15-17 or a composition of claim 18.

20. Use of at least one compound of any of claim 15-17 for the preparation of a pharmaceutical composition.

21. Use of at least one compound of any of claim 15-17 for the preparation of a pharmaceutical composition for treating and/or preventing diseases or pathologic conditions associated with cell types that express PPAR receptors.


SEQUENCE LISTING

<110> INSERM Institut National de la Santé et de la Recherche

<120> Cofactor-based screening method for nuclear receptor
      modulators and related modulators

<130> D19587

<140> US 60/297,772
<141> 2001-06-14

<160> 6

<170> PatentIn Ver. 2.1

<210> 1
<211> 1263
<212> PRT
<213> Homo sapiens

<220>
<223> TIF2 (GRIP1; NCOA-2)

<400> 1
Met Leu Val Lys Pro Leu Pro Asp Ser Glu Glu Glu Gly His Asp Asn
  1               5                  10                  15

Gln Glu Ala His Gln Lys Tyr Glu Thr Met Gln Cys Phe Ala Val Ser
             20                  25                  30

Gln Pro Lys Ser Ile Lys Glu Glu Gly Glu Asp Leu Gln Ser Cys Leu
         35                  40                  45

Ile Cys Val Ala Arg Arg Val Pro Met Lys Glu Arg Pro Val Leu Pro
     50                  55                  60

Ser Ser Glu Ser Phe Thr Thr Arg Gln Asp Leu Gln Gly Lys Ile Thr
 65                  70                  75                  80

Ser Leu Asp Thr Ser Thr Met Arg Ala Ala Met Lys Pro Gly Trp Glu
                 85                  90                  95

Asp Leu Val Arg Arg Cys Ile Gln Lys Phe His Ala Gln His Glu Gly
            100                 105                 110

Glu Ser Val Ser Tyr Ala Lys Arg His His His Glu Val Leu Arg Gln
        115                 120                 125

Gly Leu Ala Phe Ser Gln Ile Tyr Arg Phe Ser Leu Ser Asp Gly Thr
    130                 135                 140

Leu Val Ala Ala Gln Thr Lys Ser Lys Leu Ile Arg Ser Gln Thr Thr
145                 150                 155                 160

Asn Glu Pro Gln Leu Val Ile Ser Leu His Met Leu His Arg Glu Gln
                165                 170                 175

Asn Val Cys Val Met Asn Pro Asp Leu Thr Gly Gln Thr Met Gly Lys

180                 185                 190

Pro Leu Asn Pro Ile Ser Ser Asn Ser Pro Ala His Gln Ala Leu Cys
        195                 200                 205

Ser Gly Asn Pro Gly Gln Asp Met Thr Leu Ser Ser Asn Ile Asn Phe
    210                 215                 220

Pro Ile Asn Gly Pro Lys Glu Gln Met Gly Met Pro Met Gly Arg Phe
225                 230                 235                 240

Gly Gly Ser Gly Gly Met Asn His Val Ser Gly Met Gln Ala Thr Thr
                245                 250                 255

Pro Gln Gly Ser Asn Tyr Ala Leu Lys Met Asn Ser Pro Ser Gln Ser
            260                 265                 270

Ser Pro Gly Met Asn Pro Gly Gln Pro Thr Ser Met Leu Ser Pro Arg
        275                 280                 285

His Arg Met Ser Pro Gly Val Ala Gly Ser Pro Arg Ile Pro Pro Ser
    290                 295                 300

Gln Phe Ser Pro Ala Gly Ser Leu His Ser Pro Val Gly Val Cys Ser
305                 310                 315                 320

Ser Thr Gly Asn Ser His Ser Tyr Thr Asn Ser Ser Leu Asn Ala Leu
                325                 330                 335

Gln Ala Leu Ser Glu Gly His Gly Val Ser Leu Gly Ser Ser Leu Ala
            340                 345                 350

Ser Pro Asp Leu Lys Met Gly Asn Leu Gln Asn Ser Pro Val Asn Met
        355                 360                 365

Asn Pro Pro Pro Leu Ser Lys Met Gly Ser Leu Asp Ser Lys Asp Cys
    370                 375                 380

Phe Gly Leu Tyr Gly Glu Pro Ser Glu Gly Thr Thr Gly Gln Ala Glu
385                 390                 395                 400

Ser Ser Cys His Pro Gly Glu Gln Lys Glu Thr Asn Asp Pro Asn Leu
                405                 410                 415

Pro Pro Ala Val Ser Ser Glu Arg Ala Asp Gly Gln Ser Arg Leu His
            420                 425                 430

Asp Ser Lys Gly Gln Thr Lys Leu Leu Gln Leu Leu Thr Thr Lys Ser
        435                 440                 445

Asp Gln Met Glu Pro Ser Pro Leu Ala Ser Ser Leu Ser Asp Thr Asn
    450                 455                 460

Lys Asp Ser Thr Gly Ser Leu Pro Gly Ser Gly Ser Thr His Gly Thr
465                 470                 475                 480

Ser Leu Lys Glu Lys His Lys Ile Leu His Arg Leu Leu Gln Asp Ser
                485                 490                 495

Ser Ser Pro Val Asp Leu Ala Lys Leu Thr Ala Glu Ala Thr Gly Lys
            500                 505                 510

Asp Leu Ser Gln Glu Ser Ser Ser Thr Ala Pro Gly Ser Glu Val Thr
        515                 520                 525

Ile Lys Gln Glu Pro Val Ser Pro Lys Lys Lys Glu Asn Ala Leu Leu
    530                 535                 540

Arg Tyr Leu Leu Asp Lys Asp Asp Thr Lys Asp Ile Gly Leu Pro Glu
545                 550                 555                 560

Ile Thr Pro Lys Leu Glu Arg Leu Asp Ser Lys Thr Asp Pro Ala Ser
                565                 570                 575

Asn Thr Lys Leu Ile Ala Met Lys Thr Glu Lys Glu Glu Met Ser Phe
            580                 585                 590

Glu Pro Gly Asp Gln Pro Gly Ser Glu Leu Asp Asn Leu Glu Glu Ile
        595                 600                 605

Leu Asp Asp Leu Gln Asn Ser Gln Leu Pro Gln Leu Phe Pro Asp Thr
    610                 615                 620

Arg Pro Gly Ala Pro Ala Gly Ser Val Asp Lys Gln Ala Ile Ile Asn
625                 630                 635                 640

Asp Leu Met Gln Leu Thr Ala Glu Asn Ser Pro Val Thr Pro Val Gly
                645                 650                 655

Ala Gln Lys Thr Ala Leu Arg Ile Ser Gln Ser Thr Phe Asn Asn Pro
            660                 665                 670

Arg Pro Gly Gln Leu Gly Arg Leu Leu Pro Asn Gln Asn Leu Pro Leu
        675                 680                 685

Asp Ile Thr Leu Gln Ser Pro Thr Gly Ala Gly Pro Phe Pro Pro Ile
    690                 695                 700

Arg Asn Ser Ser Pro Tyr Ser Val Ile Pro Gln Pro Gly Met Met Gly
705                 710                 715                 720

Asn Gln Gly Met Ile Gly Asn Gln Gly Asn Leu Gly Asn Ser Ser Thr
                725                 730                 735

Gly Met Ile Gly Asn Ser Ala Ser Arg Pro Thr Met Pro Ser Gly Glu
            740                 745                 750

Trp Ala Pro Gln Ser Ser Ala Val Arg Val Thr Cys Ala Ala Thr Thr
        755                 760                 765

Ser Ala Met Asn Arg Pro Val Gln Gly Gly Met Ile Arg Asn Pro Ala
    770                 775                 780

Ala Ser Ile Pro Met Arg Pro Ser Ser Gln Pro Gly Gln Arg Gln Thr
785                 790                 795                 800

Leu Gln Ser Gln Val Met Asn Ile Gly Pro Ser Glu Leu Glu Met Asn
                805                 810                 815

Met Gly Gly Pro Gln Tyr Ser Gln Gln Gln Ala Pro Pro Asn Gln Thr
            820                 825                 830

Ala Pro Trp Pro Glu Ser Ile Leu Pro Ile Asp Gln Ala Ser Phe Ala
        835                 840                 845

Ser Gln Asn Arg Gln Pro Phe Gly Ser Ser Pro Asp Asp Leu Leu Cys
    850                 855                 860

Pro His Pro Ala Ala Glu Ser Pro Ser Asp Glu Gly Ala Leu Leu Asp
865                 870                 875                 880

Gln Leu Tyr Leu Ala Leu Arg Asn Phe Asp Gly Leu Glu Glu Ile Asp
                885                 890                 895

Arg Ala Leu Gly Ile Pro Glu Leu Val Ser Gln Ser Gln Ala Val Asp
            900                 905                 910

Pro Glu Gln Phe Ser Ser Gln Asp Ser Asn Ile Met Leu Glu Gln Lys
        915                 920                 925

Ala Pro Val Phe Pro Gln Gln Tyr Ala Ser Gln Ala Gln Met Ala Gln
    930                 935                 940

Gly Ser Tyr Ser Pro Met Gln Asp Pro Asn Phe His Thr Met Gly Gln
945                 950                 955                 960

Arg Pro Ser Tyr Ala Thr Leu Arg Met Gln Pro Arg Pro Gly Leu Arg
                965                 970                 975

Pro Thr Gly Leu Val Gln Asn Gln Pro Asn Gln Leu Arg Leu Gln Leu
            980                 985                 990

Gln His Arg Leu Gln Ala Gln Gln Asn Arg Gln Pro Leu Met Asn Gln
        995                 1000                1005

Ile Ser Asn Val Ser Asn Val Asn Leu Thr Leu Arg Pro Gly Val Pro
    1010                1015                1020

Thr Gln Ala Pro Ile Asn Ala Gln Met Leu Ala Gln Arg Gln Arg Glu
1025                1030                1035                1040

Ile Leu Asn Gln His Leu Arg Gln Arg Gln Met His Gln Gln Gln Gln
                1045                1050                1055

Val Gln Gln Arg Thr Leu Met Met Arg Gly Gln Gly Leu Asn Met Thr
            1060                1065                1070

Pro Ser Met Val Ala Pro Ser Gly Met Pro Ala Thr Met Ser Asn Pro
        1075                1080                1085

Arg Ile Pro Gln Ala Asn Ala Gln Gln Phe Pro Phe Pro Pro Asn Tyr
    1090                1095                1100

Gly Ile Ser Gln Gln Pro Asp Pro Gly Phe Thr Gly Ala Thr Thr Pro
1105                1110                1115                1120

Gln Ser Pro Leu Met Ser Pro Arg Met Ala His Thr Gln Ser Pro Met
                1125                1130                1135

Met Gln Gln Ser Gln Ala Asn Pro Ala Tyr Gln Ala Pro Ser Asp Ile
            1140                1145                1150

Asn Gly Trp Ala Gln Gly Asn Met Gly Gly Asn Ser Met Phe Ser Gln

1155                1160                1165

Gln Ser Pro Pro His Phe Gly Gln Gln Ala Asn Thr Ser Met Tyr Ser
    1170                1175                1180

Asn Asn Met Asn Ile Asn Val Ser Met Ala Thr Asn Thr Gly Gly Met
1185                1190                1195                1200

Ser Ser Met Asn Gln Met Thr Gly Gln Ile Ser Met Thr Ser Val Thr
                1205                1210                1215

Ser Val Pro Thr Ser Gly Leu Ser Ser Met Gly Pro Glu Gln Val Asn
            1220                1225                1230

Asp Pro Ala Leu Arg Gly Gly Asn Leu Phe Pro Asn Gln Leu Pro Gly
        1235                1240                1245

Met Asp Met Ile Lys Gln Glu Gly Asp Thr Thr Arg Lys Tyr Cys
    1250                1255                1260


<210> 2
<211> 6015
<212> DNA
<213> Homo sapiens

<220>
<223> TIF-2

<400> 2
ttgctgatat gtgttcaaga tgagtgggat gggagaaaat acctctgacc cctccagggc 60
agagacaaga aagcgcaagg aatgtcctga ccaacttgga cccagcccca aaaggaacac 120
tgaaaaacgt aatcgtgaac aggaaaataa atatatagaa gaacttgcag agttgatttt 180
tgcaaatttt aatgatatag acaactttaa cttcaaacct gacaaatgtg caatcttaaa 240
agaaactgtg aagcaaattc gtcagatcaa agaacaagag aaagcagcag ctgccaacat 300
agatgaagtg cagaagtcag atgtatcctc tacagggcag ggtgtcatcg acaaggatgc 360
gctggggcct atgatgcttg aggcccttga tgggttcttc tttgtagtga acctggaagg 420
caacgttgtg tttgtgtcag agaatgtgac acagtatcta aggtataacc aagaagagct 480
gatgaacaaa agtgtatata gcatcttgca tgttggggac cacacggaat ttgtcaaaaa 540
cctgctgcca aagtctatag gtaaatgggg gatcttggtc tggcgaacct ccgaggcgga 600
acagccatac cttcaattgt cggatgctgg taaaaccttt acctgattca gaagaggagg 660
gtcatgataa ccaggaagct catcagaaat atgaaactat gcagtgcttc gctgtctctc 720
aaccaaagtc catcaaagaa gaaggagaag atttgcagtc ctgcttgatt tgcgtggcaa 780
gaagagttcc catgaaggaa agaccagttc ttccctcatc agaaagtttt actactcgcc 840
aggatctcca aggcaagatc acgtctctgg ataccagcac catgagagca gccatgaaac 900
caggctggga ggacctggta agaaggtgta ttcagaagtt ccatgcgcag catgaaggag 960
aatctgtgtc ctatgctaag aggcatcatc atgaagtact gagacaagga ttggcattca 1020
gtcaaatcta tcgtttttcc ttgtctgatg gcactcttgt tgctgcacaa acgaagagca 1080
aactcatccg ttctcagact actaatgaac ctcaacttgt aatatcttta catatgcttc 1140
acagagagca gaatgtgtgt gtgatgaatc cggatctgac tggacaaacg atggggaagc 1200
cactgaatcc aattagctct aacagccctg cccatcaggc cctgtgcagt gggaacccag 1260
gtcaggacat gaccctcagt agcaatataa attttcccat aaatggccca aaggaacaaa 1320
tgggcatgcc catgggcagg tttggtggtt ctgggggaat gaaccatgtg tcaggcatgc 1380
aagcaaccac tcctcagggt agtaactatg cactcaaaat gaacagcccc tcacaaagca 1440
gccctggcat gaatccagga cagcccacct ccatgctttc accaaggcat cgcatgagcc 1500
ctggagtggc tggcagccct cgaatcccac ccagtcagtt ttcccctgca ggaagcttgc 1560
attcccctgt gggagtttgc agcagcacag gaaatagcca tagttatacc aacagctccc 1620
tcaatgcact tcaggccctc agcgaggggc acggggtctc attagggtca tcgttggctt 1680
caccagacct aaaaatgggc aatttgcaaa actccccagt taatatgaat cctcccccac 1740
tcagcaagat gggaagcttg gactcaaaag actgttttgg actatatggg gagccctctg 1800
aaggtacaac tggacaagca gagagcagct gccatcctgg agagcaaaag gaaacaaatg 1860

accccaacct gcccccggcc gtgagcagtg agagagctga cgggcagagc agactgcatg 1920
acagcaaagg gcagaccaaa ctcctgcagc tgctgaccac caaatctgat cagatggagc 1980
cctcgccctt agccagctct ttgtcggata caaacaaaga ctccacaggt agcttgcctg 2040
gttctgggtc tacacatgga acctcgctca aggagaagca taaaattttg cacagactct 2100
tgcaggacag cagttcccct gtggacttgg ccaagttaac agcagaagcc acaggcaaag 2160
acctgagcca ggagtccagc agcacagctc ctggatcaga agtgactatt aaacaagagc 2220
cggtgagccc caagaagaaa gagaatgcac tacttcgcta tttgctagat aaagatgata 2280
ctaaagatat tggtttacca gaaataaccc ccaaacttga gagactggac agtaagacag 2340
atcctgccag taacacaaaa ttaatagcaa tgaaaactga gaaggaggag atgagctttg 2400
agcctggtga ccagcctggc agtgagctgg acaacttgga ggagattttg gatgatttgc 2460
agaatagtca attaccacag cttttcccag acacgaggcc aggcgcccct gctggatcag 2520
ttgacaagca agccatcatc aatgacctca tgcaactcac agctgaaaac agccctgtca 2580
cacctgttgg agcccagaaa acagcactgc gaatttcaca gagcactttt aataacccac 2640
gaccagggca actgggcagg ttattgccaa accagaattt accacttgac atcacattgc 2700
aaagcccaac tggtgctgga cctttcccac caatcagaaa cagtagtccc tactcagtga 2760
tacctcagcc aggaatgatg ggtaatcaag ggatgatagg aaaccaagga aatttaggga 2820
acagtagcac aggaatgatt ggtaacagtg cttctcggcc tactatgcca tctggagaat 2880
gggcaccgca gagttcggct gtgagagtca cctgtgctgc taccaccagt gccatgaacc 2940
ggccagtcca aggaggtatg attcggaacc cagcagccag catccccatg aggcccagca 3000
gccagcctgg ccaaagacag acgcttcagt ctcaggtcat gaatataggg ccatctgaat 3060
tagagatgaa catgggggga cctcagtata gccaacaaca agctcctcca aatcagactg 3120
ccccatggcc tgaaagcatc ctgcctatag accaggcgtc ttttgccagc caaaacaggc 3180
agccatttgg cagttctcca gatgacttgc tatgtccaca tcctgcagct gagtctccga 3240
gtgatgaggg agctctcctg gaccagctgt atctggcctt gcggaatttt gatggcctgg 3300
aggagattga tagagcctta ggaatacccg aactggtcag ccagagccaa gcagtagatc 3360
cagaacagtt ctcaagtcag gattccaaca tcatgctgga gcagaaggcg cccgttttcc 3420
cacagcagta tgcatctcag gcacaaatgg cccagggtag ctattctccc atgcaagatc 3480
caaactttca caccatggga cagcggccta gttatgccac actccgtatg cagcccagac 3540
cgggcctcag gcccacgggc ctagtgcaga accagccaaa tcaactaaga cttcaacttc 3600
agcatcgcct ccaagcacag cagaatcgcc agccacttat gaatcaaatc agcaatgttt 3660
ccaatgtgaa cttgactctg aggcctggag taccaacaca ggcacctatt aatgcacaga 3720
tgctggccca gagacagagg gaaatcctga accagcatct tcgacagaga caaatgcatc 3780
agcaacagca agttcagcaa cgaactttga tgatgagagg acaagggttg aatatgacac 3840
caagcatggt ggctcctagt ggtatgccag caactatgag caaccctcgg attccccagg 3900
caaatgcaca gcagtttcca tttcctccaa actacggaat aagtcagcaa cctgatccag 3960
gctttactgg ggctacgact ccccagagcc cacttatgtc accccgaatg gcacatacac 4020
agagtcccat gatgcaacag tctcaggcca acccagccta tcaggccccc tccgacataa 4080
atggatgggc gcaggggaac atgggcggaa acagcatgtt ttcccagcag tccccaccac 4140
actttgggca gcaagcaaac accagcatgt acagtaacaa catgaacatc aatgtgtcca 4200
tggcgaccaa cacaggtggc atgagcagca tgaaccagat gacaggacag atcagcatga 4260
cctcagtgac ctccgtgcct acgtcagggc tgtcctccat gggtcccgag caggttaatg 4320
atcctgctct gaggggaggc aacctgttcc caaaccagct gcctggaatg gatatgatta 4380
agcaggaggg agacacaaca cggaaatatt gctgacactg ctgaagccag ttgcttcttc 4440
agctgaccgg gctcacttgc tcaaaacact tccagtctgg agagctgtgt ctatttgttt 4500
caacccaact gacctgccag ccggttctgc tagagcagac aggcctggcc ctggttccca 4560
gggtggcgtc cactcggctg tggcaggagg agctgcctct tctcttgaca gtctgaagct 4620
cgcatccaga cagtcgctca gtctgttcac tgcattcacc ttagtgcaac ttagatctct 4680
cctgcaaaag taaatgttga caggcaaatt tcatacccat gtcagattga atgtatttaa 4740
atgtatgtat ttaaggagaa ccatgctctt gttctgttcc tgttcggttc cagacactgg 4800
tttcttgctt tgttttccct ggctaacagt ctagtgcaaa agattaagat tttatctggg 4860
ggaaagaaaa gaatttttta aaaaattaaa ctaaagatgt tttaagctaa agcctgaatt 4920
tgggatggaa gcaggacaga caccgtggac agcgctgtat ttacagacac acccagtgcg 4980
tgaagaccaa caaagtcaca gtcgtatctc tagaaagctc taaagaccat gttggaaaga 5040
gtctccagtt actgaacaga tgaaaaggag cctgtgagag ggctgttaac attagcaaat 5100
attttttcct tgttttttct ttgttaaaac caaactggtt cacctgaatc atgaattgag 5160
aagaaataat tttcatttct aaattaagtc ccttttagtt tgatcagaca gcttgaatca 5220
gcatctcttc ttccctgtca gcctgactct tcccttcccc tctctcattc cccatactcc 5280
ctattttcat tcctttttta aaaaataata taagctacag aaaccaggta agccctttat 5340
ttccttaaat gttttgccag ccacttacca attgctaagt attgaatttc agaaaaaaaa 5400
aatgcattta ctggcaagga gaagagcaaa gttaaggctt gataccaatc gagctaagga 5460
tacctgcttt ggaagcatgt ttattctgtt ccccagcaac tctggcctcc aaaatgggag 5520

aaaacgccag tgtgtttaaa ttgatagcag atatcacgac agatttaacc tctgccatgt 5580
gtttttattt ttgtttttta gcagtgctga ctaagccgaa gttttgtaag gtacataaaa 5640
tccaatttat atgtaaacaa gcaataattt aagttgagaa cttatgtgtt ttaattgtat 5700
aatttttgtg aggtatacat attgtggaat tgactcaaaa atgaggtact tcagtattaa 5760
attagatatc ttcatagcaa tgtctcctaa aggtgttttg taaaggatat caatgccttg 5820
attagaccta atttgtagac ttaagacttt ttattttcta aaccttgtga ttctgcttat 5880
aagtcattta tctaatctat atgatatgca gccgctgtag gaaccaattc ttgattttta 5940
tatgtttata ttctttctta atgaacctta gaaagactac atgttactaa gcaggccact 6000
tttatggttg ttttt 6015

<210> 3
<211> 1441
<212> PRT
<213> Homo sapiens

<220>
<223> SRC-1. Cloning of full-length human steroid receptor
coactivator-one

<400> 3

<210> 4
<211> 4547
<212> DNA
<213> Homo sapiens

<220>
<223> SRC-1. Cloning of full-length human steroid receptor
coactivator-one

<400> 4

cggatccact agtccagtgt ggtggaattc ggcttcatca tcatgagtgg ccttggggac 60
agttcatccg accctgctaa cccagactca cataagagga aaggatcgcc atgtgacaca 120
ctggcatcaa gcacggaaaa gaggcgcagg gagcaagaaa ataaatattt agaagaacta 180
gctgagttac tgtctgccaa cattagtgac attgacagct tgagtgtaaa accagacaaa 240
tgcaagattt tgaagaaaac agtcgatcag atacagctaa tgaagagaat ggaacaagag 300
aaatcaacaa ctgatgacga tgtacagaaa tcagacatct catcaagtag tcaaggagtg 360
atagaaaagg aatccttggg acctcttctt ttggaggctt tggatggatt tttctttgtt 420
gtgaactgtg aagggagaat tgtatttgtg tcagagaatg taaccagcta cttaggttac 480
aatcaggagg aattaatgaa tacgagcgtc tacagcatac tgcacgtggg ggatcatgca 540
gaatttgtga agaatctgct accaaaatca ctagtaaatg gagttccttg gcctcaagag 600
gcaacacgac gaaatagcca tacctttaac tgcaggatgc taattcaccc tccagatgag 660
ccagggaccg agaaccaaga agcttgccag cgttatgaag taatgcagtg tttcactgtg 720
tcacagccaa aatcaattca agaggatgga gaagatttcc agtcatgtct gatttgtatt 780
gcacggcgat tacctcggcc tccagctatt acgggtgtag aatcctttat gaccaagcaa 840
gatactacag gtaaaatcat ctctattgat actagttccc tgagagctgc tggcagaact 900
ggttgggaag atttagtgag gaagtgcatt tatgcttttt tccaacctca gggcagagaa 960
ccatcttatg ccagacagct gttccaagaa gtgatgactc gtggcactgc ctccagcccc 1020
tcctatagat tcatattgaa tgatgggaca atgcttagcg cccacaccaa gtgtaaactt 1080
tgctaccctc aaagtccaga catgcaacct ttcatcatgg gaattcatat catcgacagg 1140
gagcacagtg ggctttctcc tcaagatgac actaattctg gaatgtcaat tccccgagta 1200
aatccctcgg tcaatcctag tatctctcca gctcatggtg tggctcgttc atccacattg 1260
ccaccatcca acagcaacat ggtatccacc agaataaacc gccagcagag ctcagacctt 1320
catagcagca gtcatagtaa ttctagcaac agccaaggaa gtttcggatg ctcacccgga 1380
agtcagattg tagccaatgt tgccttaaac aaaggacagg ccagttcaca gagcagtaaa 1440
ccctctttaa acctcaataa tcctcctatg gaaggtacag gaatatccct agcacagttc 1500
atgtctccaa ggagacaggt tacttctgga ttggcaacaa ggcccaggat gccaaacaat 1560
tcctttcctc ctaatatttc gacattaagc tctcccgttg gcatgacaag tagtgcctgt 1620
aataataata accgatctta ttcaaacatc ccagtaacat ctttacaggg tatgaatgaa 1680
ggacccaata actccgttgg cttctctgcc agttctccag tcctcaggca gatgagctca 1740
cagaattcac ctagcagatt aaatatacaa ccagcaaaag ctgagtccaa agataacaaa 1800
gagattgcct caactttaaa tgaaatgatt caatctgaca acagctctag tgatggcaaa 1860
cctctggatt cagggcttct gcataacaat gacagacttt cagatggaga cagtaaatac 1920
tctcaaacca gtcacaaact agtgcagctt ttgacaacaa ctgccgaaca gcagttacgg 1980
catgctgata tagacacaag ctgcaaagat gtcctgtctt gcacaggcac ttccaactct 2040
gcctctgcta actcttcagg aggttcttgt ccctcttctc atagctcatt gacagcacgg 2100
cataaaattc tacaccggct cttacaggag ggtagcccct cagatatcac cactttgtct 2160
gtcgagcctg ataaaaagga cagtgcatct acttctgtgt cagtgactgg acaggtacaa 2220
ggaaactcca gtataaaact agaactggat gcttcaaaga aaaaagaatc aaaagaccat 2280
cagctcctac gctatctttt agataaagat gagaaagatt taagatcaac tccaaacctg 2340
agcctggatg atgtaaaggt gaaagtggaa aagaaagaac agatggatcc atgtaataca 2400
aacccaaccc caatgaccaa acccactcct gaggaaataa aactggaggc ccagagccag 2460
tttacagctg accttgacca gtttgatcag ttactgccca cgctggagaa ggcagcacag 2520
ttgccaggct tatgtgagac agacaggatg gatggtgcgg tcaccagtgt aaccatcaaa 2580
tcggagatcc tgccagcttc acttcagtcc gccactgcca gacccacttc caggctgaat 2640
agattacctg agctggaatt ggaagcaatt gataaccaat ttggacaacc aggaacaggc 2700
gatcagattc catggacaaa taatacagtg acagctataa atcagagtaa atcagaagac 2760
cagtgtatta gctcacaatt agatgagctt ctctgtccac ccacaacagt agaaggagaa 2820
aatgatgaga aggctcttct tgaacagctg gtatccttcc ttagtggcaa agatgaaact 2880
gagctagctg aactagacag agctctggga attgacaaac ttgttcaggg gggtggatta 2940
gatgtattat cagagagatt tccaccacaa caagcaacgc cacctttgat catggaagaa 3000
agacccaacc tttattccca gccttactct tctccttttc ctactgccaa tctccctagc 3060
cctttccaag gcatggtcag gcaaaaacct tcactgggga cgatgcctgt tcaagtaaca 3120
cctccccgag gtgctttttc acctggcatg ggcatgcagc ccaggcaaac tctaaacaga 3180
cctccggctg cacctaacca gcttcgactt caactacagc agcgattaca gggacaacag 3240
cagttgatac accaaaatcg gcaagctatc ttaaaccagt ttgcagcaac tgctcctgtt 3300
ggcatcaata tgagatcagg catgcaacag caaattacac ctcagccacc cctgaatgct 3360
caaatgttgg cacaacgtca gcgggaactg tacagtcaac agcaccgaca gaggcagcta 3420
atacagcagc aaagagccat gcttatgagg cagcaaagct ttgggaacaa cctccctccc 3480
tcatctggac taccagttca aacggggaac ccccgtcttc ctcagggtgc tccacagcaa 3540
ttcccctatc caccaaacta tggtacaaat ccaggaaccc cacctgcttc taccagcccg 3600
ttttcacaac tagcagcaaa tcctgaagca tccttggcca accgcaacag catggtgagc 3660

agaggcatga caggaaacat aggaggacag tttggcactg gaatcaatcc tcagatgcag 3720
cagaatgtct tccagtatcc aggagcagga atggttcccc aaggtgaggc caactttgct 3780
ccatctctaa gccctgggag ctccatggtg ccgatgccaa tccctcctcc tcagagttct 3840
ctgctccagc aaactccacc tgcctccggg tatcagtcac cagacatgaa ggcctggcag 3900
caaggagcga taggaaacaa caatgtgttc agtcaagctg tccagaacca gcccacgcct 3960
gcacagccag gagtatacaa caacatgagc atcaccgttt ccatggcagg tggaaatacg 4020
aatgttcaga acatgaaccc aatgatggcc cagatgcaga tgagctcttt gcagatgcca 4080
ggaatgaaca ctgtgtgccc tgagcagata aatgatcccg cactgagaca cacaggcctc 4140
tactgcaacc agctctcatc cactgacctt ctcaaaacag aagcagatgg aacccagcag 4200
gtgcaacagg ttcaggtgtt tgctgacgtc cagtgtacag tgaatctggt aggcggggac 4260
ccttacctga accagcctgg tccactggga actcaaaagc ccacgtcagg accacagacc 4320
ccccaggccc agcagaagag cctccgtcag cagctactga ctgaataacc acttttaaag 4380
gaatgtgaaa tttaaataat agacatacag agatatacaa atatattata tatttttctg 4440
agatttttga tatctcaatc tgcagccatt cttcaggtcg tagcatttgg agcaaaaaaa 4500
aaaaaaaaaa tcgatgtcga gagtacttct agagggcccg tttaaac 4547

<210> 5
<211> 798
<212> PRT
<213> Homo sapiens

<220>
<223> PGC-1. PPAR gamma coactivator-1

<400> 5
Met Ala Trp Asp Met Cys Asn Gln Asp Ser Glu Ser Val Trp Ser Asp
1               5                   10                  15

Ile Glu Cys Ala Ala Leu Val Gly Glu Asp Gln Pro Leu Cys Pro Asp
            20                  25                  30

Leu Pro Glu Leu Asp Leu Ser Glu Leu Asp Val Asn Asp Leu Asp Thr
        35                  40                  45

Asp Ser Phe Leu Gly Gly Leu Lys Trp Cys Ser Asp Gln Ser Glu Ile
    50                  55                  60

Ile Ser Asn Gln Tyr Asn Asn Glu Pro Ser Asn Ile Phe Glu Lys Ile
65                  70                  75                  80

Asp Glu Glu Asn Glu Ala Asn Leu Leu Ala Val Leu Thr Glu Thr Leu
                85                  90                  95

Asp Ser Leu Pro Val Asp Glu Asp Gly Leu Pro Ser Phe Asp Ala Leu
            100                 105                 110

Thr Asp Gly Asp Val Thr Thr Asp Asn Glu Ala Ser Pro Ser Ser Met
        115                 120                 125

Pro Asp Gly Thr Pro Pro Pro Gln Glu Ala Glu Glu Pro Ser Leu Leu
    130                 135                 140

Lys Lys Leu Leu Leu Ala Pro Ala Asn Thr Gln Leu Ser Tyr Asn Glu
145                 150                 155                 160

Cys Ser Gly Leu Ser Thr Gln Asn His Ala Asn His Asn His Arg Ile
                165                 170                 175

Arg Thr Asn Pro Ala Ile Val Lys Thr Glu Asn Ser Trp Ser Asn Lys
            180                 185                 190

Ala Lys Ser Ile Cys Gln Gln Gln Lys Pro Gln Arg Arg Pro Cys Ser
        195                 200                 205

Glu Leu Leu Lys Tyr Leu Thr Thr Asn Asp Asp Pro Pro His Thr Lys
    210                 215                 220

Pro Thr Glu Asn Arg Asn Ser Ser Arg Asp Lys Cys Thr Ser Lys Lys
225                 230                 235                 240

Lys Ser His Thr Gln Ser Gln Ser Gln His Leu Gln Ala Lys Pro Thr
                245                 250                 255

Thr Leu Ser Leu Pro Leu Thr Pro Glu Ser Pro Asn Asp Pro Lys Gly
            260                 265                 270

Ser Pro Phe Glu Asn Lys Thr Ile Glu Arg Thr Leu Ser Val Glu Leu
        275                 280                 285

Ser Gly Thr Ala Gly Leu Thr Pro Pro Thr Thr Pro Pro His Lys Ala
    290                 295                 300

Asn Gln Asp Asn Pro Phe Arg Ala Ser Pro Lys Leu Lys Ser Ser Cys
305                 310                 315                 320

Lys Thr Val Val Pro Pro Pro Ser Lys Lys Pro Arg Tyr Ser Glu Ser
                325                 330                 335

Ser Gly Thr Gln Gly Asn Asn Ser Thr Lys Lys Gly Pro Glu Gln Ser
            340                 345                 350

Glu Leu Tyr Ala Gln Leu Ser Lys Ser Ser Val Leu Thr Gly Gly His
        355                 360                 365

Glu Glu Arg Lys Thr Lys Arg Pro Ser Leu Arg Leu Phe Gly Asp His
    370                 375                 380

Asp Tyr Cys Gln Ser Ile Asn Ser Lys Thr Glu Ile Leu Ile Asn Ile
385                 390                 395                 400

Ser Gln Glu Leu Gln Asp Ser Arg Gln Leu Glu Asn Lys Asp Val Ser
                405                 410                 415

Ser Asp Trp Gln Gly Gln Ile Cys Ser Ser Thr Asp Ser Asp Gln Cys
            420                 425                 430

Tyr Leu Arg Glu Thr Leu Glu Ala Ser Lys Gln Val Ser Pro Cys Ser
        435                 440                 445

Thr Arg Lys Gln Leu Gln Asp Gln Glu Ile Arg Ala Glu Leu Asn Lys
    450                 455                 460

His Phe Gly His Pro Ser Gln Ala Val Phe Asp Asp Glu Ala Asp Lys
465                 470                 475                 480

Thr Gly Glu Leu Arg Asp Ser Asp Phe Ser Asn Glu Gln Phe Ser Lys
                485                 490                 495

Leu Pro Met Phe Ile Asn Ser Gly Leu Ala Met Asp Gly Leu Phe Asp

            500                 505                 510

Asp Ser Glu Asp Glu Ser Asp Lys Leu Ser Tyr Pro Trp Asp Gly Thr
        515                 520                 525

Gln Ser Tyr Ser Leu Phe Asn Val Ser Pro Ser Cys Ser Ser Phe Asn
    530                 535                 540

Ser Pro Cys Arg Asp Ser Val Ser Pro Pro Lys Ser Leu Phe Ser Gln
545                 550                 555                 560

Arg Pro Gln Arg Met Arg Ser Arg Ser Arg Ser Phe Ser Arg His Arg
                565                 570                 575

Ser Cys Ser Arg Ser Pro Tyr Ser Arg Ser Arg Ser Arg Ser Pro Gly
            580                 585                 590

Ser Arg Ser Ser Ser Arg Ser Cys Tyr Tyr Tyr Glu Ser Ser His Tyr
        595                 600                 605

Arg His Arg Thr His Arg Asn Ser Pro Leu Tyr Val Arg Ser Arg Ser
    610                 615                 620

Arg Ser Pro Tyr Ser Arg Arg Pro Arg Tyr Asp Ser Tyr Glu Glu Tyr
625                 630                 635                 640

Gln His Glu Arg Leu Lys Arg Glu Glu Tyr Arg Arg Glu Tyr Glu Lys
                645                 650                 655

Arg Glu Ser Glu Arg Ala Lys Gln Arg Glu Arg Gln Arg Gln Lys Ala
            660                 665                 670

Ile Glu Glu Arg Arg Val Ile Tyr Val Gly Lys Ile Arg Pro Asp Thr
        675                 680                 685

Thr Arg Thr Glu Leu Arg Asp Arg Phe Glu Val Phe Gly Glu Ile Glu
    690                 695                 700

Glu Cys Thr Val Asn Leu Arg Asp Asp Gly Asp Ser Tyr Gly Phe Ile
705                 710                 715                 720

Thr Tyr Arg Tyr Thr Cys Asp Ala Phe Ala Ala Leu Glu Asn Gly Tyr
                725                 730                 735

Thr Leu Arg Arg Ser Asn Glu Thr Asp Phe Glu Leu Tyr Phe Cys Gly
            740                 745                 750

Arg Lys Gln Phe Phe Lys Ser Asn Tyr Ala Asp Leu Asp Ser Asn Ser
        755                 760                 765

Asp Asp Phe Asp Pro Ala Ser Thr Lys Ser Lys Tyr Asp Ser Leu Asp
    770                 775                 780

Phe Asp Ser Leu Leu Lys Glu Ala Gln Arg Ser Leu Arg Arg
785                 790                 795


<210> 6
<211> 505
<212> PRT
<213> Homo sapiens

<220>
<223> PPAR-gamma. Peroxisome proliferator activated receptor
gamma

<400> 6